# EUROPEAN PATENT APPLICATION

(11) **EP 3 032 444 A1**
(43) Date of publication of application: **15.06.2016**
(21) Application number: 15172681.7
(22) Date of filing: 18.06.2015
(51) Int. Cl.: G06F 19/00

(54) **IMAGING APPARATUS AND CONTROL METHOD THEREOF**

(30) Priority: 12.12.2014 KR 20140179739
(71) Applicant: Samsung Medison Co., Ltd., Gangwon-do 250-870 (KR)
(72) Inventor: Yang, Sun-Mo, Gyeonggi-do (KR)
(74) Representative: Frey, Sven Holger

(57) **Abstract**

Disclosed herein are an imaging apparatus and a control method thereof. The imaging apparatus includes: a main body; a display unit configured to display one or more operation menus for controlling the main body; and a processor configured to control, if a virtual keyboard call command is received, the display unit to display a virtual keyboard and a sub menu related to the operation menus, instead of the operation menus.

## Description

### BACKGROUND

### 1. Field

Embodiments of the present disclosure relate to an imaging apparatus and a control method thereof.

### 2. Description of the Related Art

An imaging apparatus is an apparatus of acquiring images about the inside or outside of an object using Free Induction Decay (FID) signals that are induced by visible light, infrared light, ultrasonic waves, radiation, or a nuclear magnetic resonance phenomenon. The imaging apparatus includes a camera, an ultrasonic imaging apparatus, a radiation imaging apparatus, and a Magnetic Resonance Imaging (MRI) apparatus. The ultrasonic imaging apparatus acquires ultrasound images about the inside of an object, for example, various tissues or structures inside a human body, using ultrasonic waves. The ultrasonic imaging apparatus receives ultrasonic waves reflected from a subject or generated by a subject according to incidence of laser, and acquires ultrasound images corresponding to the received ultrasonic waves. The ultrasound images include slice images of a subject, such as slice images about soft tissue or images about blood flow. The ultrasonic imaging apparatus has advantages that it is a compact, low-priced apparatus compared to other imaging apparatuses and it can photograph 2Dimensional (2D) or 3Dimensional (3D) images in real time. Also, the ultrasonic imaging apparatus has high safety since there is no risk for patients to be exposed to radiation such as X-rays. For the advantages, the ultrasonic imaging apparatus is widely used in medical fields or in nondestructive inspection fields.

### SUMMARY

Therefore, it is an aspect of the present disclosure to provide an imaging apparatus for enabling a user to easily check and execute operation menus even when a virtual keyboard overlaps the operation menus so that the user cannot see the operation menus, and a control method of the imaging apparatus.

Additional aspects of the disclosure will be set forth in part in the description which follows and, in part, will be obvious from the description, or may be learned by practice of the disclosure.

In accordance with one aspect of the present disclosure, an imaging apparatus includes: a main body; a display unit configured to display one or more operation menus for controlling the main body; and a processor configured to control, if a virtual keyboard call command is received, the display unit to display a virtual keyboard and a sub menu related to the operation menus, instead of the operation menus.

The processor may control the display unit to display the virtual keyboard that blocks the entire or a part of the operation menus.

The sub menu may be displayed above or beside the virtual keyboard, or overlap the virtual keyboard.

The sub menu may include all of one or more functions or commands corresponding to the operation menus.

The sub menu may include one or more functions or commands having high frequency of use by a user among one or more functions or commands corresponding to the operation menus.

The sub menu may include one or more functions or commands related to an application being executed on the main body upon manipulation, among one or more functions or commands corresponding to the operation menus.

When the virtual keyboard is called, the processor may control the display unit to display information acquired in real time by the main body.

The virtual keyboard may be called according to a user's manipulation or a predetermined setting.

The imaging apparatus may further include an input unit configured to receive the virtual keyboard call command.

The display unit may include a touch screen configured to receive a command according to a touch operation.

The main body may include at least one of a visible light camera, an infrared camera, a camcorder, an ultrasonic imaging apparatus, a digital radiation imaging apparatus, a computerized tomography (CT) apparatus, a Mammography apparatus, and a Magnetic Resonance Imaging (MRI) apparatus.

At least one of the operation menus and the sub menu may further include a Time Gain Compensation (TGC) area.

The main body may further include a second display unit configured to display an image acquired by the main body.

In accordance with one aspect of the present disclosure, a method of controlling an imaging apparatus including a main body and a display unit, includes: at the display unit, displaying one or more operation menus for controlling the main body; calling a virtual keyboard; and at the display unit, displaying, if the virtual keyboard is called, the virtual keyboard and a sub menu related to the operation menus, instead of the operation menus.

The virtual keyboard may block the entire or a part of the operation menus.

The displaying, if the virtual keyboard is called, the virtual keyboard and the sub menu related to the operation menus, instead of the operation menus, may include at the display unit, displaying the sub menu above or beside the virtual keyboard or overlapping the sub menu with the virtual keyboard.

The method may further include deciding the sub menu according to a user's selection or a predetermined setting.

The deciding of the sub menu according to the user's selection or the predetermined setting may include deciding all of one or more functions corresponding to the operation menus or one or more commands related to the functions, as the sub menu.

The deciding of the sub menu according to the user's selection or the predetermined setting may include deciding one or more functions or commands having high frequency of use by the user among one or more functions or commands corresponding to the operation menus, as the sub menu.

The deciding of the sub menu according to the user's selection or the predetermined setting may include deciding one or more functions or commands related to an application being executed on the main body upon manipulation, among one or more functions or commands corresponding to the operation menus, as the sub menu.

The displaying, if the virtual keyboard is called, the virtual keyboard and the sub menu related to the operation menus, instead of the operation menus, may include displaying information acquired in real time by the main body.

The calling of the virtual keyboard may include calling the virtual keyboard according to a user's manipulation or a predetermined setting.

The display unit may include a touch screen configured to receive a command according to a touch operation.

The main body may include an imaging apparatus which is at least one of a visible light camera, an infrared camera, a camcorder, an ultrasonic imaging apparatus, a digital radiation imaging apparatus, a computerized tomography (CT) apparatus, a Mammography apparatus, and a Magnetic Resonance Imaging (MRI) apparatus.

The imaging apparatus may further include a second display unit, and the method may further include at the second display unit, displaying an image acquired by the main body.

At least one of the operation menus and the sub menu may further include a Time Gain Compensation (TGC) area.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a block diagram of an imaging apparatus according to an embodiment of the present disclosure;
FIG. 2 shows an example of a screen that is displayed on a first display unit;
FIG. 3 shows an embodiment of an operation menu screen;
FIG. 4 shows an embodiment of a virtual keyboard screen;
FIG. 5 shows an embodiment of a virtual keyboard that is displayed on a screen;
FIG. 6 shows a first embodiment of a sub menu that is displayed together with a virtual keyboard;
FIG. 7 shows a second embodiment of a sub menu;
FIG. 8 shows a third embodiment of a sub menu;
FIG. 9 shows a fourth embodiment of a sub menu;
FIG. 10 shows a real-time information display area according to an embodiment of the present disclosure;
FIGS. 11A, 11 B, and 11C are views for describing a method of displaying a virtual keyboard when the virtual keyboard is called, according to an embodiment of the present disclosure;
FIGS. 12A, 12B, and 12C are views for describing a method of displaying a virtual keyboard and a sub menu when the virtual keyboard is called, according to another embodiment of the present disclosure;
FIG. 13 shows a second embodiment of a display screen of an imaging apparatus when a virtual keyboard is called;
FIG. 14 shows a third embodiment of a display screen of an imaging apparatus when a virtual keyboard is called;
FIG. 15 shows an external appearance of an ultrasonic imaging apparatus according to an embodiment of the present disclosure;
FIG. 16 is a block diagram of an ultrasonic imaging apparatus according to an embodiment of the present disclosure;
FIG. 17 is a cross-sectional view of an ultrasound probe according to an embodiment of the present disclosure;
FIG. 18 is a view for describing a beamforming process;
FIG. 19 shows an input unit, a first display unit implemented as a touch screen, and a second display unit of an ultrasonic imaging apparatus according to an embodiment of the present disclosure;
FIG. 20 is a flowchart illustrating a method of controlling an imaging apparatus, according to an embodiment of the present disclosure; and
FIG. 21 is a flowchart illustrating a method of controlling an ultrasonic imaging apparatus, according to an embodiment of the present disclosure.

### DETAILED DESCRIPTION

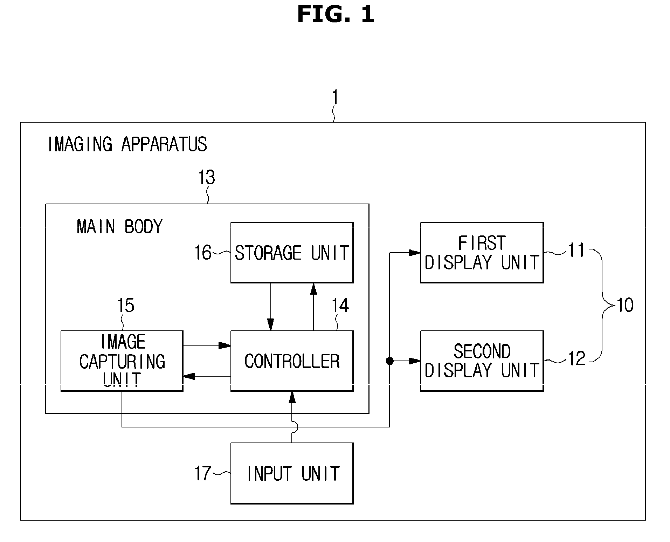

Hereinafter, an imaging apparatus according to an embodiment of the present disclosure will be described with reference to FIGS. 1 to 14.

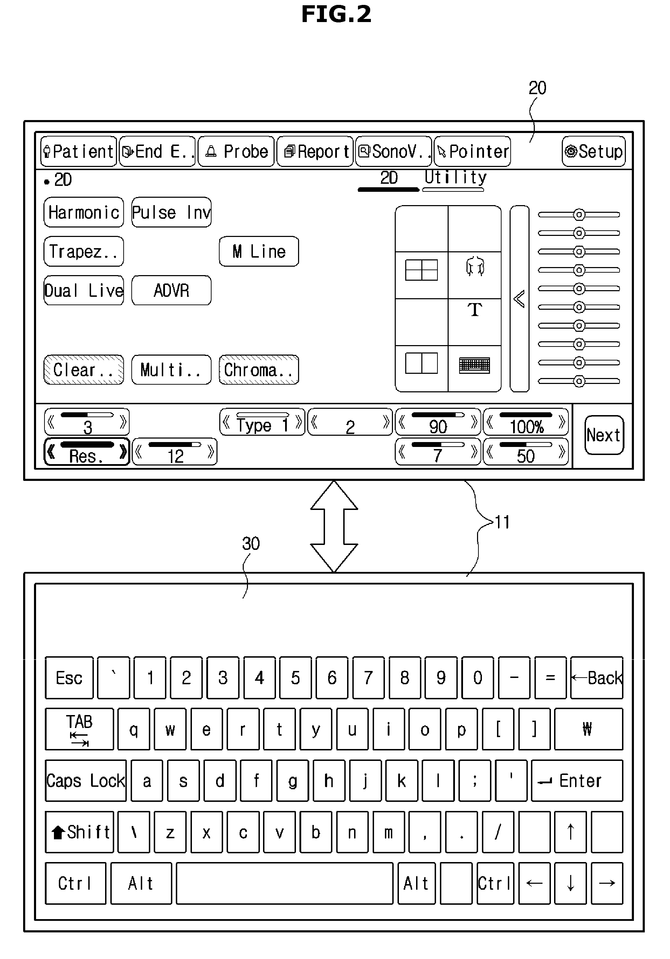

FIG. 1 is a block diagram of an imaging apparatus according to an embodiment of the present disclosure, and FIG. 2 shows an example of a screen that is displayed on a first display unit.

As shown in FIG. 1, an imaging apparatus 1 may include a display unit 10 to display images, and a main body 13 to acquire images. The display unit 10 and the main body 13 may be physically connected to each other through a cable to transmit/receive data, or may transmit/receive data through a wireless communication network. Transmission/reception of data may be performed by transmitting/receiving electrical signals corresponding to data to be transferred.

The display unit 10 may display predetermined images. Herein, the images may mean visual information that can be displayed on a 2Dimensional (2D) or 3Dimensional (3D) screen. As shown in FIG. 1, the display unit 10 may include a plurality of display units, that is, a first display unit 11 and a second display unit 12. The first display unit 11 may be physically separated from the second display unit 12.

The first display unit 11 may display an operation menu screen 20 or a virtual keyboard screen 30, wherein the operation menu screen 20 includes various virtual buttons needed to control the display unit 10 or the main body 13. Accordingly, a user can control the display unit 10 or the main body 13 through the first display unit 11.

The second display unit 12 may display an ultrasound image or a radiation image acquired by the main body 13.

The first display unit 11 and the second display unit 12 may be implemented using various kinds of displays. For example, the first display unit 11 and the second display unit 12 may be implemented using a Cathode Ray Tube (CRT), a Plasma Display Panel (PDP), Light Emitting Diodes (LEDs), a Liquid Crystal Display (LCD), a a Quantum Dot LED (QD-LED) display, or E-Ink. Also, the first display unit 11 and the second display unit 12 may be implemented using the same kind of displays or different kinds of displays.

At least one of the first display unit 11 and the second display unit 12 may be a touch screen. The touch screen is an input unit configured to receive a predetermined instruction from a user according to an input of touching the surface. The touch screen may be at least one of a resistive touch screen that determines a touch input and a touch position according to physical pressure, a capacitive touch screen that determines a touch input and a touch position using changes of electrical signals according to a touch operation, and an infrared touch screen that determines a touch input and a touch position according to whether infrared light is blocked. However, at least one of the first display unit 11 and the second display unit 12 may be any other kind of touch screen that can be considered by one of ordinary skill in the art.

Hereinafter, an example of a display screen that is displayed on the first display unit 11 will be described with reference to FIGS. 2 to 14.

The first display unit 11 may display the operation menu screen 20 as shown in FIG. 2. At this time, if a user inputs a virtual keyboard call command, or if a predetermined condition is satisfied, the first display unit 11 may display the virtual keyboard screen 30, instead of the operation menu screen 20. Also, if the user inputs an operation menu call command, or if another predetermined condition is satisfied, the first display unit 11 may display the operation menu screen 20, instead of the virtual keyboard screen 30.

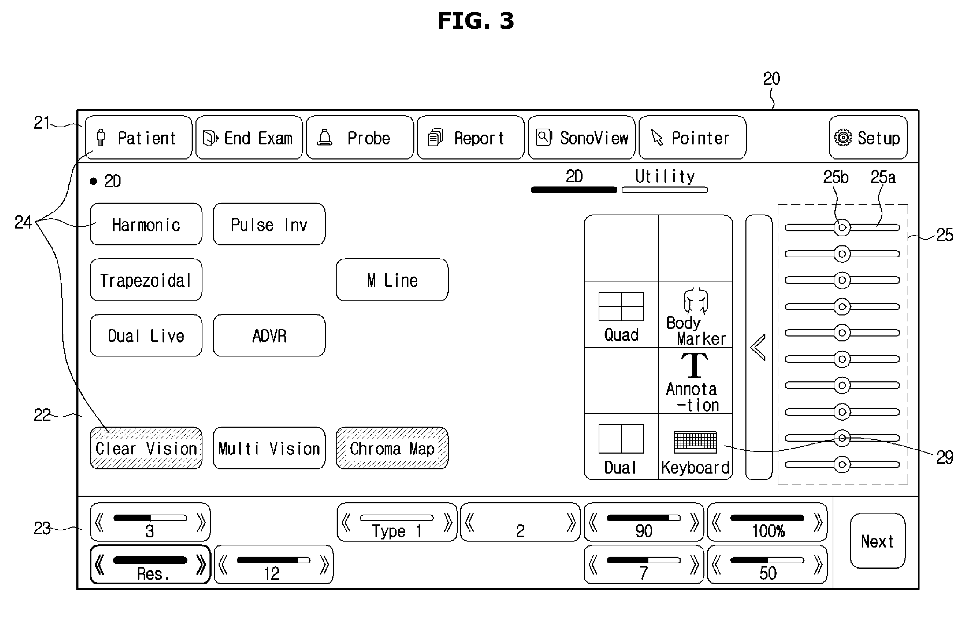

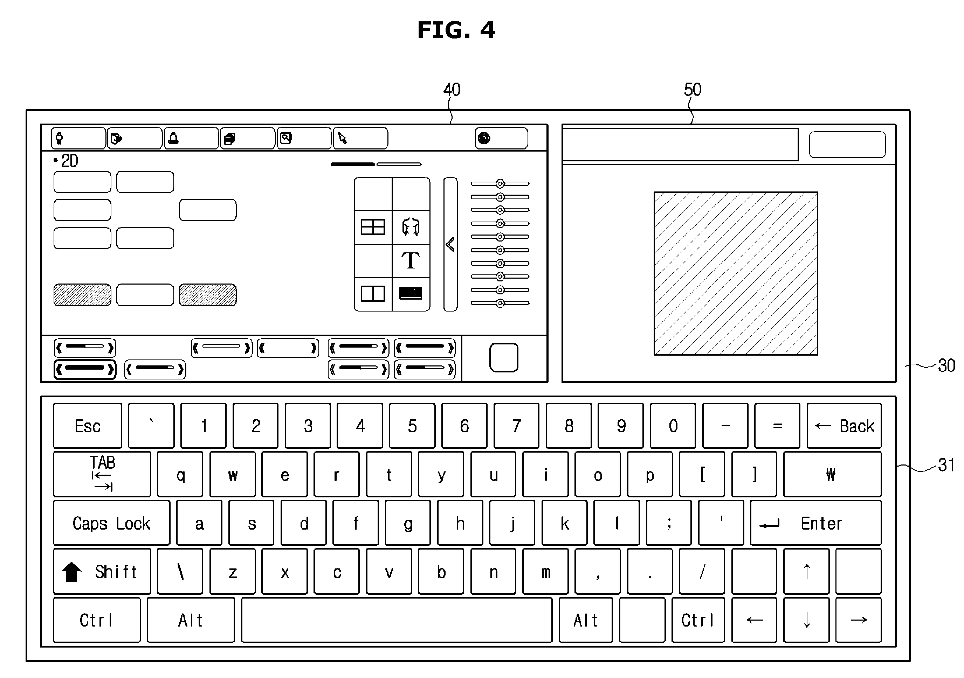

The operation menu screen 20 may be a screen in which a plurality of operation menus 24 (see FIG. 3) for controlling the imaging apparatus 1 are arranged, and the virtual keyboard screen 30 may be a screen in which a virtual keyboard 31 (see FIG. 4) is displayed at the entire area or a part. The operation menus 24 and the virtual keyboard 31 will be described in more detail later.

FIG. 3 shows an embodiment of the operation menu screen 20.

The operation menu screen 20 may display the plurality of operation menus 24 related to control operations of the main body 13. The operation menus 24 may correspond to a list of functions that are provided to a user in order to allow the user to control the display unit 10 or the main body 13, or may correspond to a list of commands related to the functions.

The operation menus 24 may include a plurality of selection areas corresponding to the individual functions or commands, in order to allow the user to select at least one from among the functions or commands.

The functions or commands may depend on the kind of the main body 13. For example, if the main body 13 is an ultrasonic imaging apparatus, the functions of the operation menus 24 may include various functions related to the ultrasonic imaging apparatus or various commands related to the functions, such as a command for instructing an ultrasound probe to irradiate ultrasonic waves, a command for inputting a patient's information to the ultrasonic imaging apparatus, a command for deciding and selecting input information, or a command for selecting a mode for ultrasound images.

The selection areas of the operation menus 24 may be implemented as images including symbols, characters, numerals, or various figures. The user may select at least one from among such symbols, characters, numerals, and figures to thereby select a desired function/command from among the functions or commands. For example, the selection areas may be represented as predetermined images functioning as Graphic User Interfaces (GUIs). In the following description, the predetermined images representing the functions or commands of the operation menus 24 will be referred to as guide images. In other words, guide images, which are GUIs, are images that are displayed on a screen in order to enable a user to easily control the corresponding apparatus.

The guide images may be configured with characters, symbols, numerals, and various figures. Also, the guide images may include various kinds of GUIs, such as virtual buttons (for example, icons), scroll bars, or track bars. Also, the guide images may be represented as images of physical input units, such as a wheel, a jog, or a knob. That is, the guide images may be decided in various shapes according to a designer's arbitrary selection or taste.

The user may select a guide image from the guide images by moving a focus or a cursor to the corresponding guide image or touching the corresponding guide image. Then, at least one function or command corresponding to the selected guide image may be input to the imaging apparatus 1.

According to an embodiment, the operation menus 24 may include a Time Gain Compensation (TGC) area 25. If the main body 13 is an ultrasonic imaging apparatus, ultrasonic waves irradiated into a subject may be reflected from a plurality of target regions having different depths. The reflected ultrasonic waves may be received by ultrasonic elements (for example, ultrasonic transducers) installed in a probe of the ultrasonic imaging apparatus. The reflected ultrasonic waves may have attenuated at different degrees of attenuation according to the depths of the target regions, which may cause differences in amplitude between the reflected ultrasonic waves. TGC is to equalize differences in amplitude between reflected ultrasonic waves according to the depths of various target regions.

The TGC area 25 may be configured with one or more adjusting bars 25a and one or more moving objects 25b that can move along the adjusting bars 25a, as shown in FIG. 3. The adjusting bars 25a and the moving objects 25b may be embodied as predetermined figures. The user may move each moving object 25b along the corresponding adjusting bar 25a by touching and dragging the moving object 25b on the screen or by locating a mouse cursor at the moving object 25b and then dragging the moving object 25b, in order to perform TGC. In FIG. 3, an embodiment in which the TGC area 25 includes the adjusting bars 25a and the moving objects 25b is shown, however, the TGC area 25 may be embodied in any other shape. For example, the TGC area 25 may be embodied by displaying characters or numerals representing various values related to TGC on the screen.

The operation menu screen 20 may be sectioned into a plurality of areas 21, 22, and 23.

According to an embodiment, the plurality of operation menus 24 that can be displayed on the screen 20 may be classified in a hierarchical structure. In this case, each area 21, 22, or 23 of the operation menu screen 20 may display one or more operation menus of a predetermined layer. For example, the plurality of areas 21, 22, and 23 may include a upper-layer display area 21 and a lower-layer display area 22, wherein the upper-layer display area 21 may display one or more upper-layer operation menus, and the lower-layer display area 22 may display one or more lower-layer operation menus corresponding to a selected upper-layer operation menu.

More specifically, the upper-layer display area 21 may display one or more relatively upper-layer operation menus related to control operations of the main body 13. For example, the upper-layer operation menus may be configured with an operation menu for selecting or changing at least one application that can be executed by the main body 13, and an operation menu for selecting a component (for example, an ultrasound probe) of the main body 13. The operation menus that are displayed in the upper-layer display area 21 may change according to the properties of the imaging apparatus 1 or according to a designer's arbitrary selection.

The lower-layer display area 22 may display one or more lower-layer operation menus corresponding to a selected upper-layer operation menu. For example, if the user selects an operation menu for manipulating the ultrasound probe from the upper-layer operation menus, the lower-layer display area 22 may display various operation menus needed for manipulating the ultrasound probe, for example, an operation menu for changing the frequency of ultrasonic waves, an operation menu for deciding weights that are applied upon beamforming, or an operation menu for setting a display mode of an A mode, a M mode, a C mode, etc. The operation menus that are displayed in the lower-layer display area 22 may depend on the selected upper-layer operation menu. The operation menus that are displayed in the lower-layer display area 22 may also change according to the properties of the imaging apparatus 1 or according to the designer's arbitrary selection.

According to an embodiment, the operation menu screen 20 may further include a setting display area 23. The setting display area 23 may display various information about the main body 13. For example, the setting display area 23 may display various setting information such as the operation environment of the main body 13.

In at least one of the upper-layer display area 21, the lower-layer display area 22, and the setting display area 23, a guide image 29 to which a virtual keyboard call function has been allocated may be provided. The user may input a virtual keyboard call command to the imaging apparatus 1 by manipulating the input unit 17 (see FIG. 1), such as a mouse or a physical button, to move a focus or a cursor to the guide image 29 or by touching the guide image 29. Then, the imaging apparatus 1 may display the virtual keyboard screen 30 (see FIG. 2) on the first display unit 11 (see FIG. 1), in response to the virtual keyboard call command, thereby displaying a virtual keyboard 31 (see FIG. 4). However, according to another embodiment, the guide image 29 may be omitted. In this case, the user may manipulate a predetermined physical button to input a virtual keyboard call command to the imaging apparatus 1.

According to an embodiment, the operation menu screen 20 may be configured by forming various characters, symbols, or images on one or more layers. The layers mean virtual 2Dimensional (2D) planes on which characters, symbols, or images can be inserted or arranged in various ways according to the designer's selection. A screen may be formed with one layer, by overlapping a plurality of layers, or by arranging a plurality of layers side by side. Each layer may be transparent except for areas in which images, etc. are displayed.

FIG. 4 shows an embodiment of the virtual keyboard screen 30.

The virtual keyboard screen 30 may display the virtual keyboard 31. The virtual keyboard screen 30 may be configured with a combination of one or more layers. The virtual keyboard screen 30 may include the virtual keyboard 31 and a sub menu 40, as shown in FIG. 4. According to an embodiment, the virtual keyboard screen 30 may further include a real-time information display area 50.

The virtual keyboard 31, the sub menu 40, and the real-time information display area 50 of the virtual keyboard screen 30 may be arranged in various ways according to the designer's selection. For example, in the embodiment of FIG. 4, the sub menu 40 may be displayed in the left upper part of the virtual keyboard screen 30, the real-time information display area 50 may be displayed in the right upper part of the virtual keyboard screen 30, and the virtual keyboard 31 may be displayed below the sub menu 40 and the real-time information display area 50.

Hereinafter, the virtual keyboard 31, the sub menu 40, and the real-time information display area 50, which are displayed in the virtual keyboard screen 30, will be described in more detail.

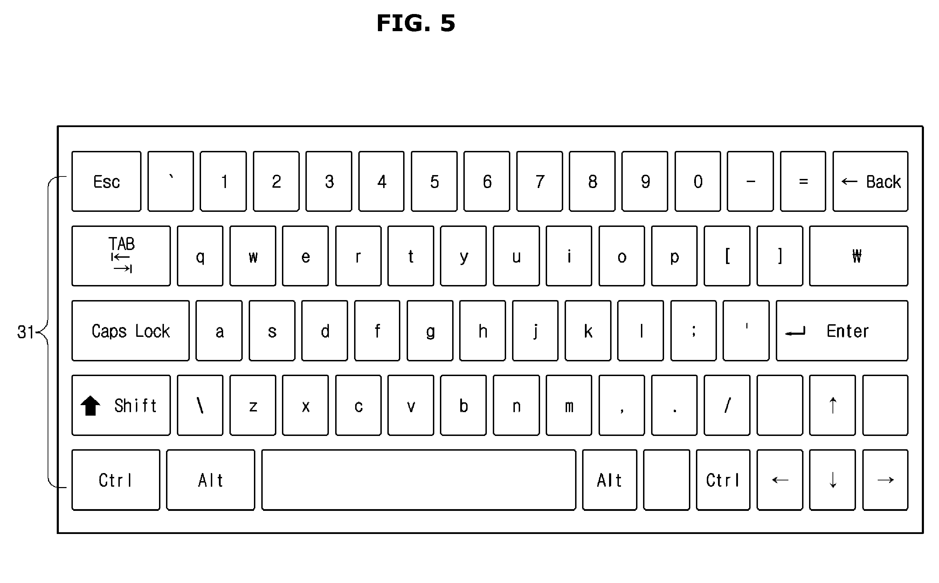

FIG. 5 shows an embodiment of a virtual keyboard that is displayed on a screen.

Referring to FIGS. 4 and 5, the virtual keyboard screen 30 may display the virtual keyboard 31. The virtual keyboard 31 may be an image in which characters, numerals, symbols, etc. are arranged. The virtual keyboard 31 may perform a function which is the same as or similar to a physical keyboard, according to a user's manipulations. Also, the virtual keyboard 31 may have a format which is the same as or similar to a physical keyboard. If the virtual keyboard 31 is displayed on the first display unit 11 (see FIG. 1), the user may move a focus or a cursor to a desired key in the virtual keyboard 31 or touch the desired key in the virtual keyboard 31 to thereby input a character, a numeral, or a symbol or to input a predetermined command.

As shown in FIG. 5, the virtual keyboard 31 may include a plurality of virtual keys to which characters, numerals, or symbols have been allocated, wherein the characters may include various formats of characters, such as Alphabets, Hangul characters, Katakana characters, or Arabic characters, which can be used as keyboard keys. If a user selects a desired key, a character, a numeral, or a symbol allocated to the selected key may be input to the imaging apparatus 1.

Also, the virtual keyboard 31 may further include a function key for inputting a predetermined command. The function key may include an enter key or a direction key. The user may input a predetermined command, for example, a command allocated to the enter key or a command allocated to the direction key, using the same method of inputting a character, a numeral, or a symbol.

The keys of the virtual keyboard 31 may be arranged in various ways. For example, the virtual keyboard 31 may be implemented as a virtual Qwerty keyboard, as shown in FIG. 4. According to another embodiment, the virtual keyboard 31 may be implemented as a virtual Dvorak keyboard. Also, according to still another embodiment, the virtual keyboard 31 may be implemented as a virtual Cheonjiin keyboard. Also, the virtual keyboard 31 may be implemented with any other keyboard arrangement that can be considered by one of ordinary skill in the art.

Each key of the virtual keyboard 31 may have one of various shapes, such as a square, a rectangle, a diamond, and a circle. Each key of the virtual keyboard 31 may be implemented in a shape corresponding to each key of a physical keyboard.

The virtual keyboard 31 may be positioned at a location decided by a system designer in the virtual keyboard screen 30. For example, the virtual keyboard 31 may be positioned at the lower part of the virtual keyboard screen 30, as shown in FIG. 4. Also, the virtual keyboard 31 may be positioned in the right or left part of the virtual keyboard screen 30. Also, the virtual keyboard 31 may be positioned in the upper part of the virtual keyboard screen 30.

When the virtual keyboard screen 30 is displayed on the first display unit 11, the virtual keyboard 31 may overlap the entire or a part of the operation menus 24 (see FIG. 3). In other words, when the first display unit 11 displays the virtual keyboard screen 30, the first display unit 11 may not display the entire or a part of the operation menu screen 20.

The sub menu 40 may be a list of functions that are provided to the user when the operation menu screen 20 is not displayed or cannot be displayed. The meaning that the sub menu 40 is associated with the operation menus 24 is that the functions of the sub menu 40 are associated with the functions of the operation menus 24. For example, the functions of the sub menu 40 may be the same as the entire or a part of the functions of the operation menus 24.

The sub menu 40 may also be represented as a plurality of guide images functioning as GUIs. As described above, each guide image may be allocated at least one command or function. A user may move a focus or a cursor to a desired guide image or touch the desired guide image to select the desired guide image, thereby inputting at least one command to the imaging apparatus 1 or selecting a function of the imaging apparatus 1.

The guide images of the sub menu 40 may be sectioned by dividing lines such that they can be distinguished from the virtual keyboard 31 or other displayed elements.

According to an embodiment, the sub menu 40 may be positioned above the virtual keyboard 31, as shown in FIG. 4. In this case, the sub menu 40 may not block the virtual keyboard 31. The size of the sub menu 40 may be smaller than that of the virtual keyboard 21.

The user may use the sub menu 40 to select the entire or a part of the functions of the operation menus 24 that are displayed on the operation menu screen 20.

Hereinafter, various embodiments of the sub menu 40 will be described.

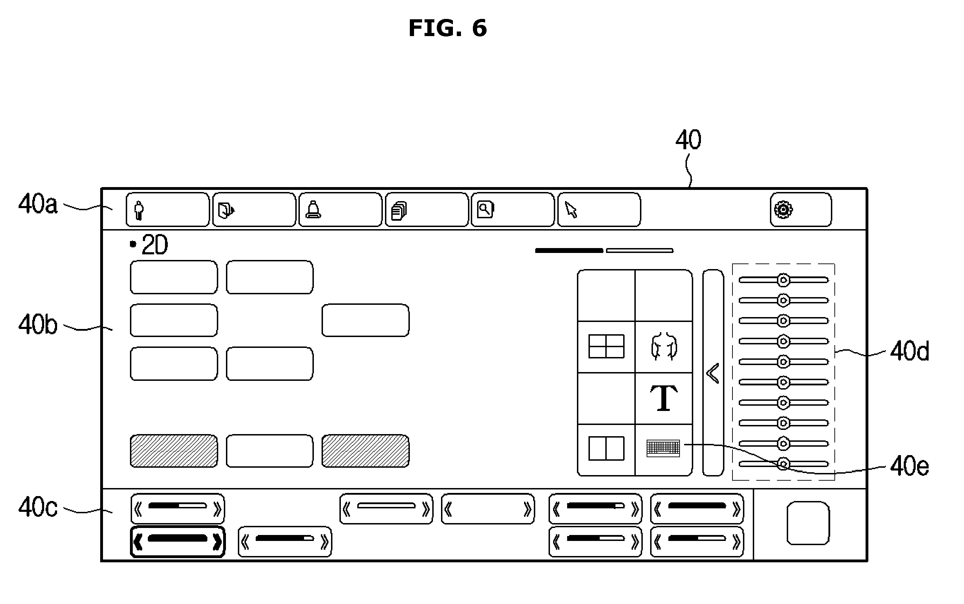

FIG. 6 shows a first embodiment of a sub menu that is displayed together with a virtual keyboard.

Referring to FIG. 6, a sub menu 40 according to a first embodiment may be configured with the same commands and functions as those of the operation menu screen 20. In this case, the sub menu 40 may have the same design as the operation menu screen 20. For example, as shown in FIG. 6, the sub menu 40 may include, like the operation menu screen 20, a upper-layer display area 40a, a lower-layer display area 40b, and a setting display area 40c. The upper-layer display area 40a may display one or more relatively upper-layer operation menus related to control operations of the main body 13. The lower-layer display area 40b may display one or more lower-layer operation menus corresponding to a selected upper-layer operation menu, and the setting display area 40c may display various information about the main body 13. In this case, the sub menu 40 may be a scaled-down version of the operation menu screen 20.

According to an embodiment, the sub menu 40 may include a TGC area 40d, like the operation menus 24 of the operation menu screen 20. As described above, the TGC area 40d may be configured with one or more adjusting bars and one or more moving objects. According to another embodiment, the TGC area 40d may be embodied by displaying characters or numerals representing various values related to TGC on the screen.

Also, the sub menu 40 may further include a virtual keyboard removal button 40e for making the virtual keyboard 31 disappear from the screen. If the user manipulates the virtual keyboard removal button 40e, the virtual keyboard 31 may disappear from the screen. The user may manipulate the virtual keyboard removal button 40e by touching the virtual keyboard removal button 40e, by locating a cursor at the virtual keyboard removal button 40, or by locating the cursor at the virtual keyboard removal button 40 and then manipulating a physical button.

According to another embodiment, the guide images of the operation menu screen 20, to which predetermined functions have been allocated, may be different from guide images of the sub menu 40, to which the same functions have been allocated. The reason is because guide images may be distorted to lower visibility due to low resolution, etc. when the operation menu screen 20 is scaled down.

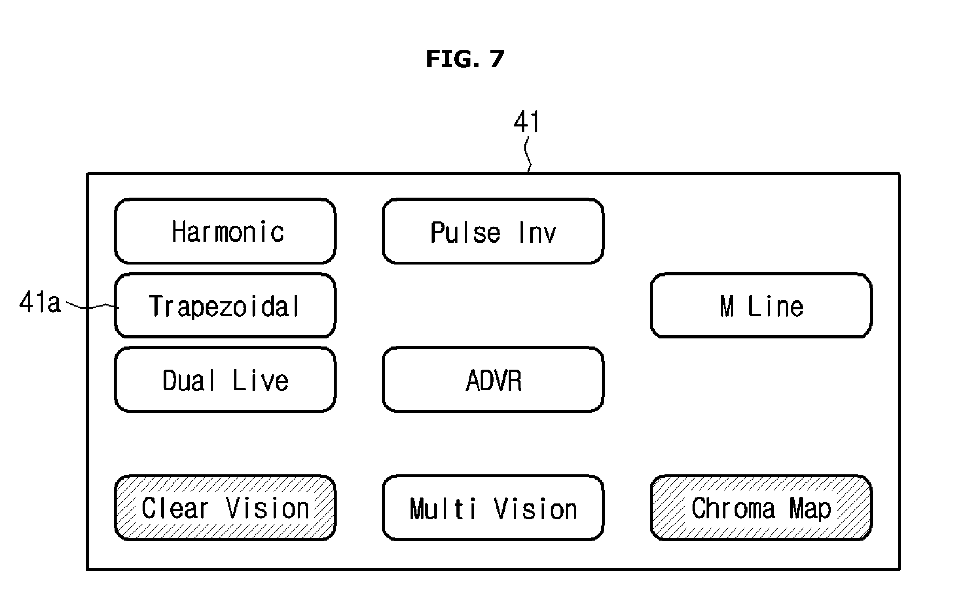

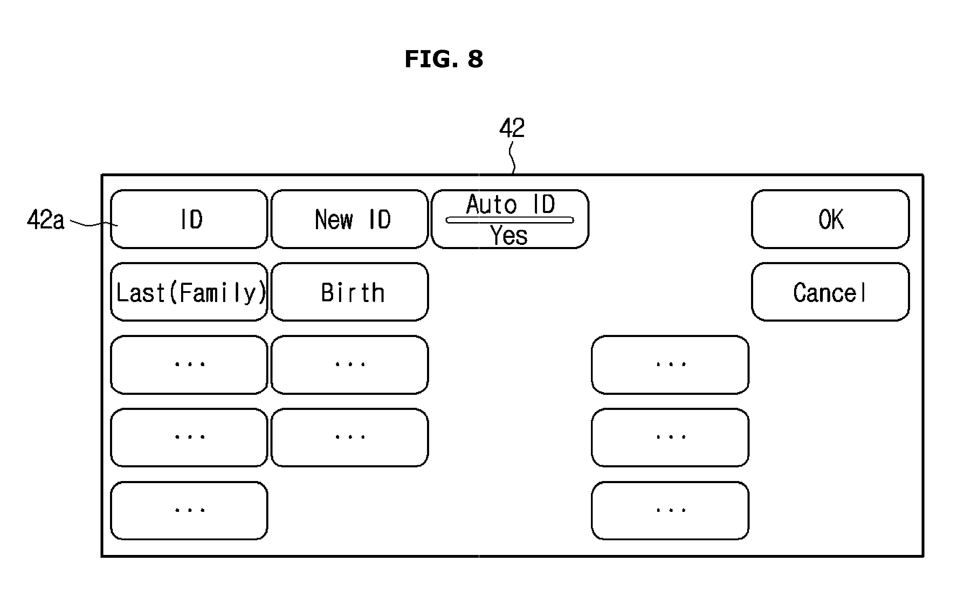

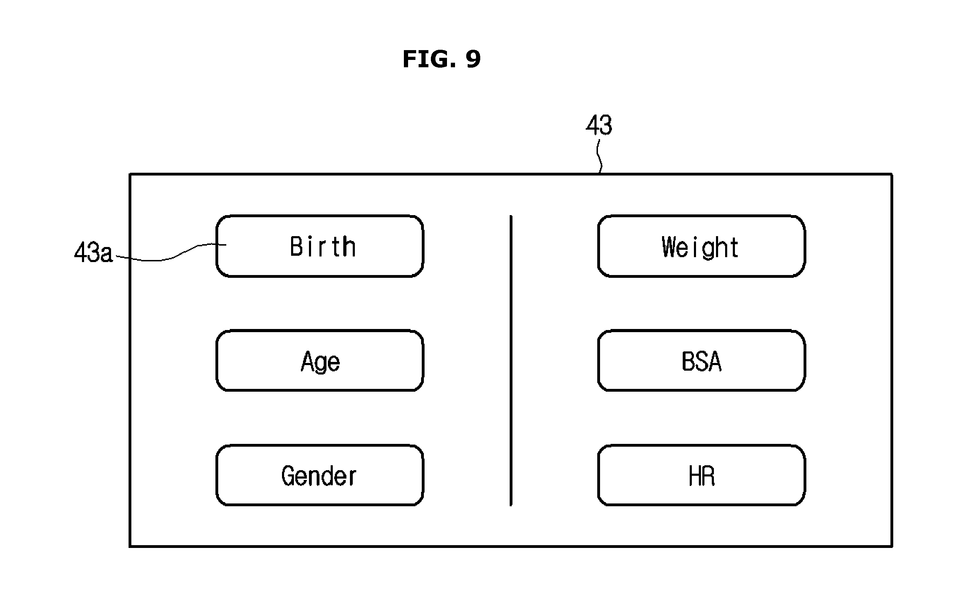

FIG. 7 shows a second embodiment of a sub menu, FIG. 8 shows a third embodiment of a sub menu, and FIG. 9 shows a fourth embodiment of a sub menu.

Referring to FIGS. 7, 8, and 9, sub menus 41, 42, and 43 according to the second, third, and fourth embodiments may be configured with a part of the functions of the operation menus 24 (see FIG. 3). Referring to FIG. 7, the sub menu 41 may include one or more functions selected by a user or according to a predetermined setting from among the functions of the operation menus 24.

The sub menus 41, 42, and 43 may display one or more guide images 41 a, 42a, and 43a to enable the user to select a desired function.

When the sub menus 41, 42, and 43 are configured with a part of the functions of the operation menus 24, the guide image 41 a, 42a, or 43a corresponding to a function may be the same as or different from the corresponding guide image of the operation menu screen 20. Meanwhile, when the sub menus 41, 42, and 43 are configured with a part of the functions of the operation menus 24, the guide image 41 a, 42a, or 43a corresponding to a function may be designed with a relatively larger size since the smaller number of functions are displayed on a screen.

The sub menus 41 and 42 may include a function corresponding to an application being executed when the virtual keyboard is called.

For example, as shown in FIG. 7, if an image capturing application for controlling image capturing of the imaging apparatus 1 has been being executed when the virtual keyboard is called, the sub menu 41 may be configured with various functions related to image capturing. In this case, the sub menu 41 may include one or more functions that are displayed in the lower-layer display area 20b of the operation menu screen 20.

As another example, as shown in FIG. 8, if an examinee management application for inputting data about a examinee, such as a patient's name or resident registration number, to the imaging apparatus 1 has been being executed when the virtual keyboard is called, the sub menu 42 may be configured with one or more functions related with inputs of data about a examinee, such as an identification number, a family name, a name, an age, a gender, a height, a weight, the date of birth, a Body Surface Area (BSA), and a Heart Rate (HR).

Also, referring to FIG. 9, the sub menu 43 may be configured with one or more functions having high frequency of use or expected to have high frequency of use, among the functions of the operation menus 24.

According to an embodiment, the sub menu 43 may be configured with a predetermined number of functions that a user relatively often has selected for a predetermined time period from a manipulation time. According to another embodiment, the sub menu 43 may be configured with one or more functions having high frequency of selection among a plurality of functions that can be performed by an application being executed when the virtual keyboard is called. According to still another embodiment, the sub menu 43 may be configured with one or more functions having high frequency of use when the virtual keyboard is called among the plurality of functions of the operation menus 24.

For example, when the examinee management application is executed and the virtual keyboard is called, the user may mainly use functions of inputting the date of birth, an age, a gender, a weight, a BSA, and a HR among the functions related to inputs of data about a examinee. In this case, the sub menu 43 may be configured with the functions of inputting the date of birth, an age, a gender, a weight, a BSA, and a HR.

The number of functions included in the sub menu 43 may depend on the user's selection or a predetermined setting. The number of functions included in the sub menu 43 may be smaller than the numbers of functions included in the sub menus 41 and 42 as shown in FIGS. 7 and 8.

The sub menu 40 may be positioned on the same layer as the virtual keyboard 31 or on a layer that is different from that of the virtual keyboard 31. The sub menu 40 may also be configured with a combination of one or more layers.

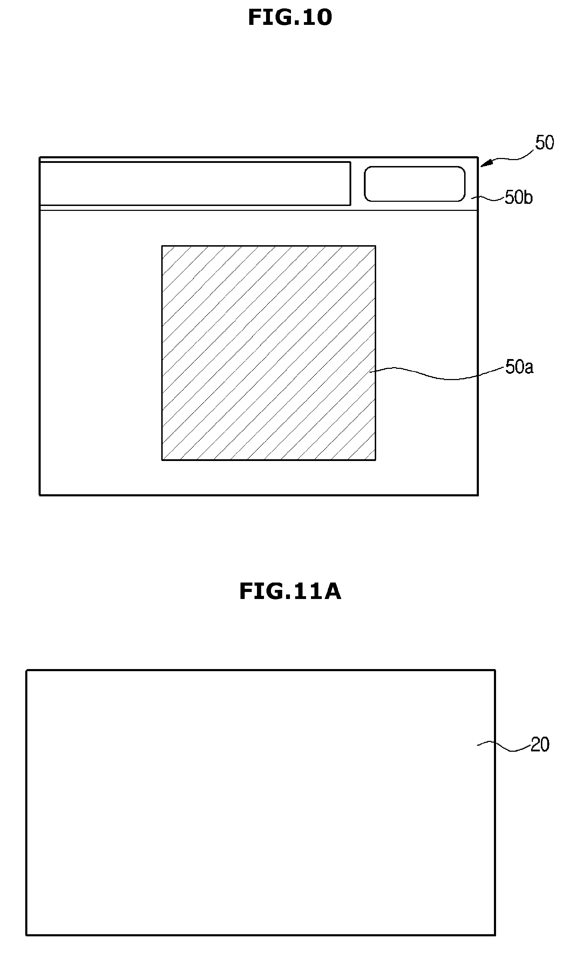

FIG. 10 shows a real-time information display area according to an embodiment of the present disclosure.

Referring to FIG. 4, the real-time information display area 50 may be displayed in an area in which the virtual keyboard 31 and the sub menu 40 are not displayed.

The real-time information display area 50 may provide a function of displaying various information acquired in real time when the imaging apparatus 1 operates. The real-time information display area 50 may display information acquired in real time using characters, numerals, or symbols, or using still images or moving images. In order to display information acquired in real time using characters, numerals, or symbols, or using still images or moving images, the real-time information display area 50 may include a display window 50a for displaying characters, numerals, symbols, still images, or moving images, as shown in FIG. 10.

For example, if the user selects a guide image corresponding to a body marker to input the body marker while inputting a character using the virtual keyboard, the display window 50a of the real-time information display area 50 may display the body marker in real time so that the user can easily adjust the body marker in real time.

The real-time information display area 50 may further include a command input part 50b. The command input part 50b may include a guide image to display or not display the real-time information display area 50, to receive a command for changing content that is displayed on the display window 50a, or to receive various commands related to the real-time information display area 50.

The real-time information display area 50 may be positioned on the same layer as the virtual keyboard 31 or the sub menu 40, or on a layer that is different from that of the virtual keyboard 31 or the sub menu 40. The real-time information display area 50 may be configured with a combination of one or more layers.

However, the real-time information display area 50 may be omitted.

Hereinafter, an embodiment of a method of displaying a virtual keyboard or both the virtual keyboard and a sub menu when the virtual keyboard is called will be described with reference to FIGS. 11A to 12C.

In FIGS. 11A to 12C, an area surrounded by thick lines is a display screen that is displayed on the first display unit 11 (see FIG. 1), and areas outside the thick lines are not displayed on the screen. However, for convenience of description, in FIGS. 11A to 12C, the remaining areas other than the display screen are also shown.

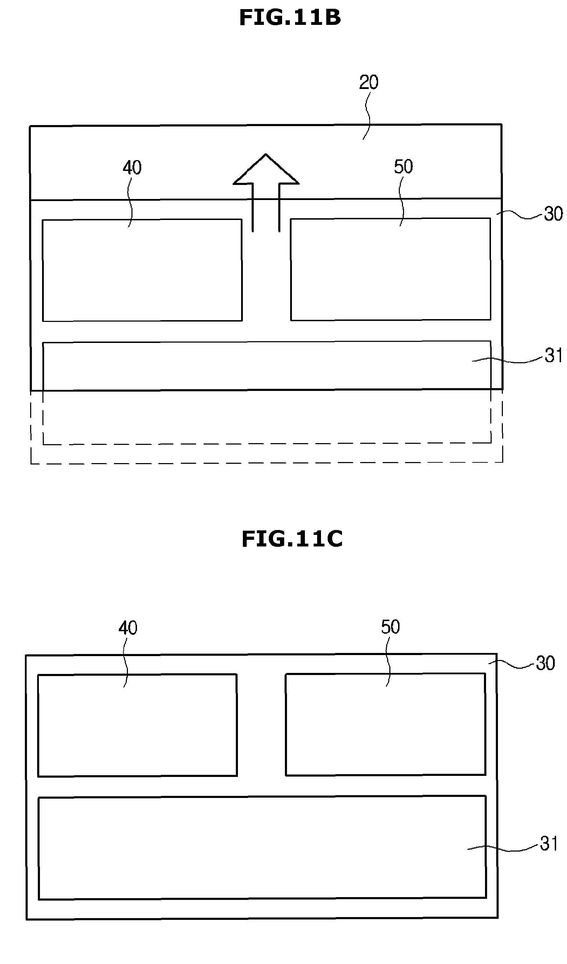

FIGS. 11A, 11 B, and 11C are views for describing a method of displaying a virtual keyboard when the virtual keyboard is called, according to an embodiment of the present disclosure.

As shown in FIG. 11A, before a command for calling a virtual keyboard is received, the first display unit 11 (see FIG. 1) may display only the operation menu screen 20. At this time, a user may input a command for controlling the imaging apparatus 1 using operation menus of the operation menu screen 20.

According to an embodiment, if a command for calling a virtual keyboard is input by the user, the first display unit 11 may display the virtual keyboard screen 30, as shown in FIGS. 11 B and 11C. The user may input a command for calling a virtual keyboard by using the guide image 29 for calling a virtual keyboard, or by manipulating separate input means, for example, a physical button. According to another embodiment, the virtual keyboard screen 30 may be displayed according to a predetermined setting. For example, when a situation needing to input a character, a numeral, or a symbol using the virtual keyboard occurs in manipulating the imaging apparatus 1, the imaging apparatus 1 may call the virtual keyboard automatically. Accordingly, the first display unit 11 may display the virtual keyboard screen 30.

If a command of calling a virtual keyboard is input, the virtual keyboard 31 and the sub menu 40 may appear on the first display unit 11 from below and move to the upper screen area of the first display unit 11 to gradually change the operation menu screen 20 to the virtual keyboard screen 30, as shown in FIG. 11B.

The virtual keyboard 31 and the sub menu 40 may move together in the same direction at the same speed. For example, the virtual keyboard 31 and the sub menu 40 may move from the lower screen area to the upper screen area to block the entire or a part of a layer corresponding to the operation menu screen 20.

Finally, as shown in FIG. 11C, the virtual keyboard screen 30 and the sub menu 40 may stop at a predetermined position. More specifically, the virtual keyboard screen 30 and the sub menu 40 may stop at a predetermined position at which the virtual keyboard screen 30 and the sub menu 40 completely block the operation menu screen 20. The predetermined position may be the upper end of the screen.

Meanwhile, the real-time information display area 50 may also move at the same speed as the virtual keyboard 31 and the sub menu 40 in the same direction as the virtual keyboard 31 and the sub menu 40, as shown in FIG. 11B.

According to another embodiment, the sub menu 40 may be called and displayed according to a separate command such as a user's command for calling a sub menu. In other words, the sub menu 40 may be not called when the virtual keyboard 31 is called, but called according to a separate command before or after the virtual keyboard 31 is called. In this case, the operation menu screen 20 or the virtual keyboard screen 30 may further include a guide screen for calling the sub menu 40.

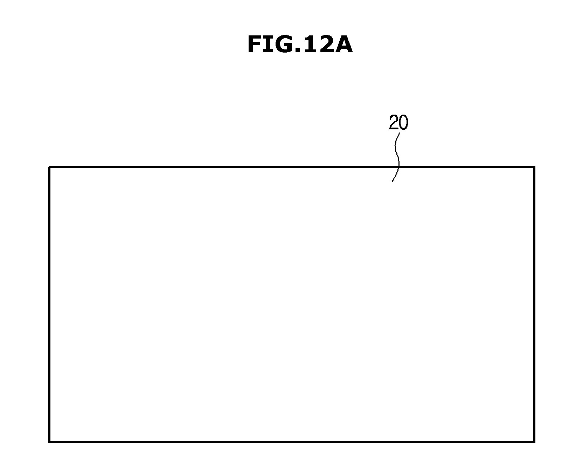

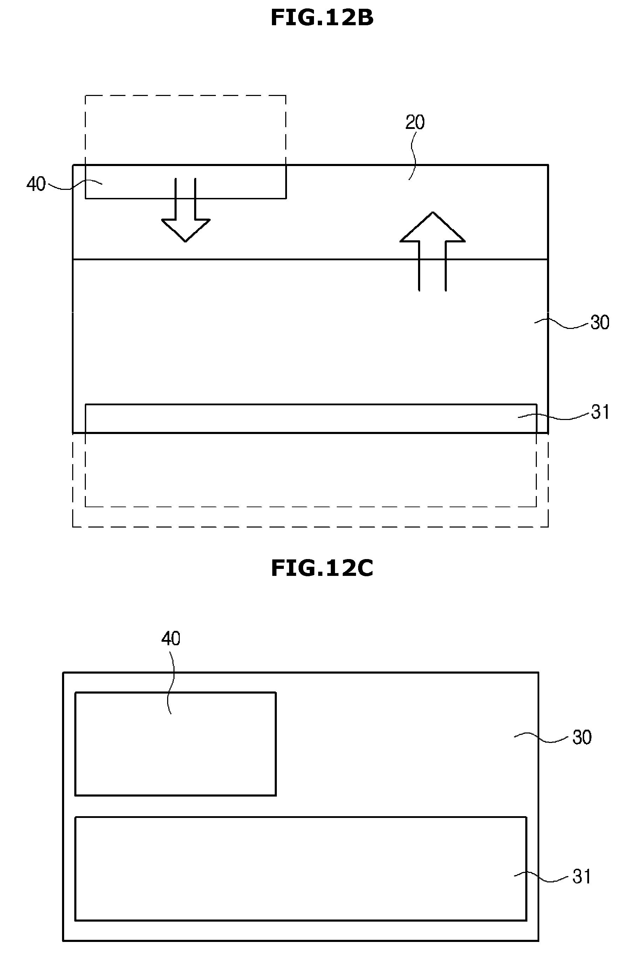

FIGS. 12A, 12B, and 12C are views for describing a method of displaying a virtual keyboard and a sub menu when the virtual keyboard is called, according to another embodiment of the present disclosure.

As shown in FIGS. 12A and 12B, if a command for calling a virtual keyboard is input when the operation menu screen 20 is displayed, the virtual keyboard 31 may appear from below and move to the upper screen area, and the sub menu 40 may appear from above and move to the lower screen area in a direction that is opposite to the movement direction of the virtual keyboard 31, thereby displaying the virtual keyboard screen 30. In this case, the sub menu 40 may stop at a part of the virtual keyboard screen 30, for example, at the left upper area or the right upper area of the virtual keyboard31. The real-time information display area 50 may also move in the same direction as the sub menu 40, and stop at the left upper area or the right upper area of the virtual keyboard screen 30. Likewise, the sub menu 40 may also be called and displayed according to a separate command such as a user's command of calling a sub menu, as described above.

Various examples in which the first display unit 11 changes the operation menu screen 20 to the virtual keyboard screen 30 have been described above. However, there may be various methods of changing the operation menu screen 20 to the virtual keyboard screen 30.

For example, the virtual keyboard screen 30 may be displayed by moving the virtual keyboard 31 and the sub menu 40 from the right of the screen to the left. Also, the virtual keyboard screen 30 may be displayed by moving the virtual keyboard 31 from the right of the screen to the left and the sub menu 40 from the left of the screen to the right. Also, the virtual keyboard 31 and the sub menu 40 may be displayed using a screen conversion method such as dissolve. Also, the virtual keyboard 31 and the sub menu 40 may be displayed without using a separate screen conversion method. Also, the first display unit 11 may display the virtual keyboard screen 30 using various methods that can be considered by one of ordinary skill in the art.

The first display unit 11 may change the virtual keyboard screen 30 to the operation menu screen 20 according to the user's selection or a predetermined setting. In this case, the predetermined setting may be the elapse of a predetermined time.

The virtual keyboard screen 30 may disappear in the reverse order from that as shown in FIGS. 11A to 12C. For example, the virtual keyboard 31 and the sub menu 40 may move to the lower area of the screen to gradually expose the operation menu screen 20 on the screen, and then may disappear from the screen.

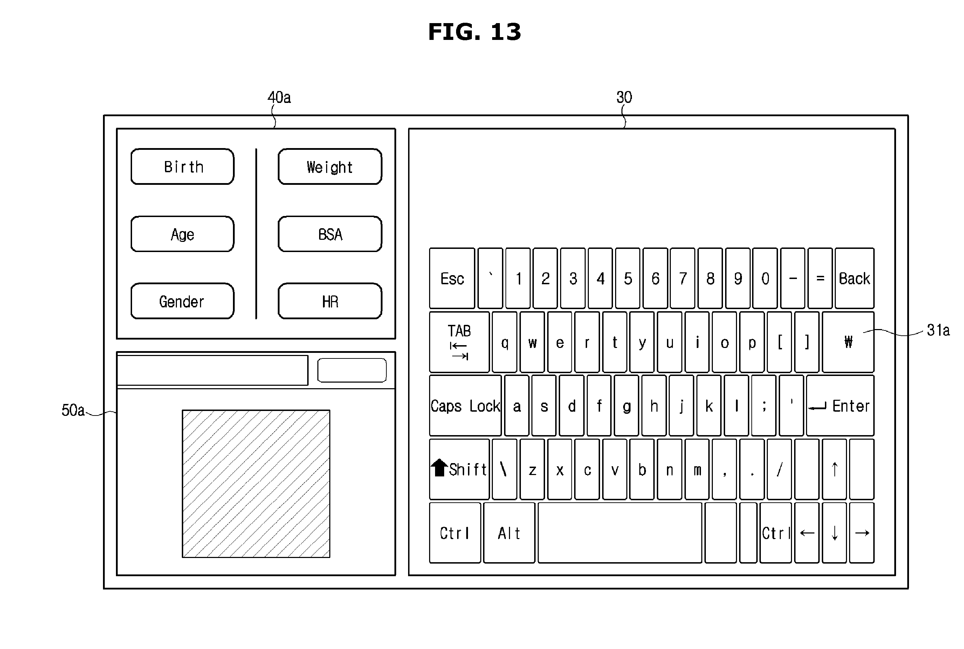

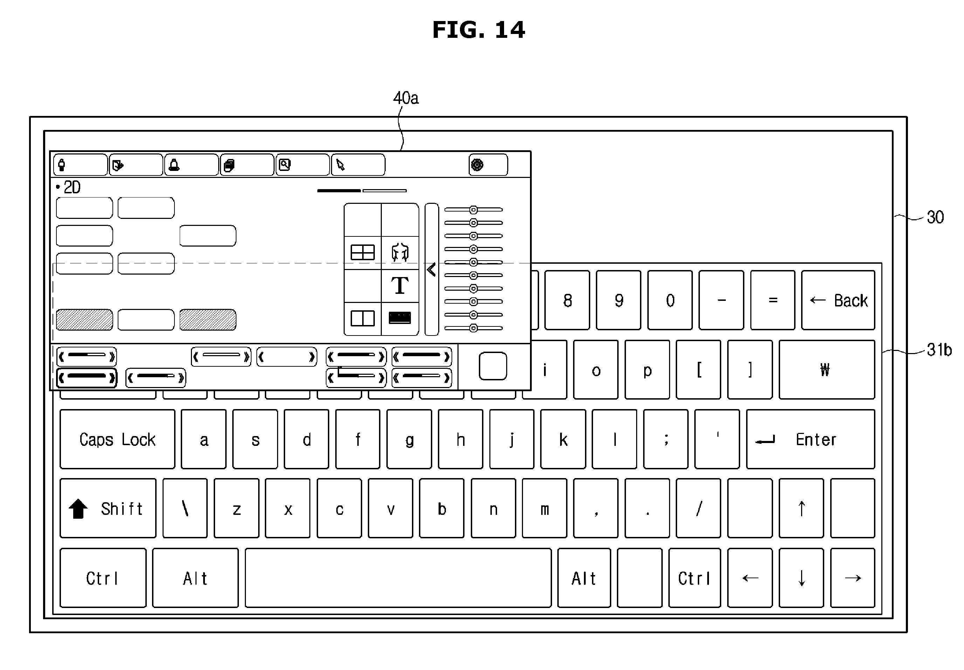

Hereinafter, another example related to arrangement of the virtual keyboard 31, the sub menu 40, and the real-time information display area 50 on the virtual keyboard screen 30 will be described with reference to FIGS. 13 and 14.

FIG. 13 shows a second embodiment of a display screen of the imaging apparatus 1 when the virtual keyboard is called, and FIG. 14 shows a third embodiment of a display screen of the imaging apparatus 1 when the virtual keyboard is called.

Unlike the screen shown in FIG. 4, a sub menu 40a may be positioned at the left upper part of a virtual keyboard screen 30, a real-time information display area 50a may be positioned at the left lower part of the virtual keyboard screen 30, and a virtual keyboard 31 a may be positioned to the right of the sub menu 40a and the display window 50a, as shown in FIG. 13. In this case, the virtual keyboard 31 a may appear from the right of the screen, move to the left of the screen, and stop at the position shown in FIG. 13, while the sub menu 40 and the real-time information display area 50a may appear from the left of the screen, move in a direction that is opposite to the movement direction of the virtual keyboard 31 a, and stop at the position shown in FIG. 13.

According to another embodiment as shown in FIG. 14, a sub menu 40b( 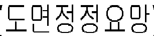 ) may overlap a virtual keyboard 31 b. In other words, the sub menu 40b may block a part of the virtual keyboard 31 b. In this case, the sub menu 40b may overlap the virtual keyboard 31 b according to a user's command for calling a sub menu, and then, disappear from the screen according to a user's selection or a predetermined setting for manipulation of the virtual keyboard 31 b. The predetermined setting may be the elapse of a predetermined time. In other words, the sub menu 40b may be displayed or not displayed on the screen according to a user's manipulation.

An example of a display screen that is displayed on the first display unit 11 has been described. The display screen that is displayed on the first display unit 11 may be displayed on the second display unit 12. In other words, the second display unit 12 may display, like the first display unit 11, operation menus, a virtual keyboard, a sub menu, etc. According to another embodiment, the first display unit 11 and the second display unit 12 may display different menus. For example, the first display unit 11 may display a part of functions of operation menus, and the second display unit 12 may display the other part of the functions of the operation menus.

Also, the second display unit 12 may display various images, such as ultrasound images or radiation images, which are provided by the imaging apparatus 1. For example, when the first display unit 11 displays an operation menu or a virtual keyboard, the second display unit 12 may display an image, such as a ultrasound image or a radiation image, which cannot be displayed in the first display unit 11. Accordingly, a user can examine images provided by the imaging apparatus 1 while manipulating the imaging apparatus 1 using the operation menu or the virtual keyboard displayed on the first display unit 11.

Referring again to FIG. 1, the main body 13 of the imaging apparatus 1 may include a controller 14, an image capturing unit 15, and a storage unit 16.

The controller 14 may control the entire operations of the imaging apparatus 1. More specifically, the controller 14 may decide an image that is displayed on the first display unit 11 according to a user's instruction input through the input unit 17 or according to a predetermined setting, and transfer the result of the decision in the form of an electrical signal to the first display unit 11, so that the first display unit 11 can display an image according to the result of the decision.

More specifically, the controller 14 may control the first display unit 11 to selectively output the operation menu screen 20 or the virtual keyboard screen 30. For example, the controller 14 may enable the virtual keyboard 31 to be displayed on the first display unit 11 according to a user's selection or a predetermined setting. Also, the controller 14 may decide a kind of a sub menu that is to be displayed on the first display unit 11, and cause a sub menu to be displayed on the first display unit 11, according to the result of the decision.

The controller 14 may be implemented as a Central Processing Unit (CPU) or a Graphic Processing Unit (GPU) provided in an external housing in which various elements of the imaging apparatus 1 are installed or in a separate workstation connected to the imaging apparatus 1.

The image capturing unit 15 may capture images about a subject. According to an embodiment, the image capturing unit 15 may acquire images about a subject or correct acquired images using visible light, infrared light, ultrasonic waves, radiation, or FID signals. According to a method in which the image capturing unit 15 acquires images, the main body 13 may be classified into one among a visible light camera, an infrared camera, a camcorder, an ultrasonic imaging apparatus, a digital radiation imaging apparatus, a computerized tomography (CT) apparatus, a Mammography apparatus, and a Magnetic Resonance Imaging (MRI) apparatus. An image acquired by the image capturing unit 15 may be displayed on the second display unit 12.

The storage unit 16 may store various programs or related information associated with processing of functions of the controller 14, or images that are to be displayed on the first display unit 11 or the second display unit 12 and information about the images. The storage unit 16 may store various data required to display the operation menu screen 20 and the virtual keyboard screen 30. For example, the storage unit 16 may store data about a layer corresponding to the operation menu screen 20 and a layer corresponding to the virtual keyboard 31. Also, the storage unit 16 may further store the real-time information display area 50 and various data related to the real-time information display area 50. The storage unit 16 may be implemented as a semiconductor storage device, a magnetic disk storage device, or a magnetic tape storage device.

As shown in FIG. 1, the imaging apparatus 1 may further include the input unit 17. The input unit 17 may receive various commands for controlling the imaging apparatus 1 from a user. The input unit 17 may output an input signal according to a user's manipulation, and transfer the input signal to the controller 14. The input unit 17 may be coupled with the main body 13 of the imaging apparatus 1, or may be separated from the main body 13. When the input unit 17 is separated from the main body 13, the input unit 17 may connect to the main body 13 through a separate cable to communicate with the main body 13, or may connect to the main body 13 through a wireless communication network such as Bluetooth to communicate with the main body 13.

The input unit 17 may include a mouse, a physical keyboard, a physical button, a track ball, a touch pad, a stick type manipulation unit, or a knob. However, the input unit 17 may be any other input device that can be considered by one of ordinary skill in the art. According to another embodiment, the input unit 17 may be omitted. For example, when the first display unit 4 is a touch screen, the input unit 17 may be omitted.

The user may use the input unit 17 to select at least one screen 20, 30 and/or 40 that is to be displayed on the first display unit 11. For example, if the user manipulates a button of the input unit 17 to input a command for displaying a virtual keyboard, the controller 14 may output a command for calling a virtual keyboard so that the virtual keyboard screen 20 is displayed on the first display unit 11.

Also, the user may use the input unit 17 to select guide images of the operation menu screen 20 and the sub menu 40, or to select a virtual key of the virtual keyboard 31 provided on the virtual keyboard screen 30. For example, the user may manipulate a mouse or a track ball to move a cursor to a guide image in the operation menu screen 20, and then manipulate a separate physical button to select a function corresponding to the guide image. Then, the imaging apparatus 1 may operate according to the selected function.

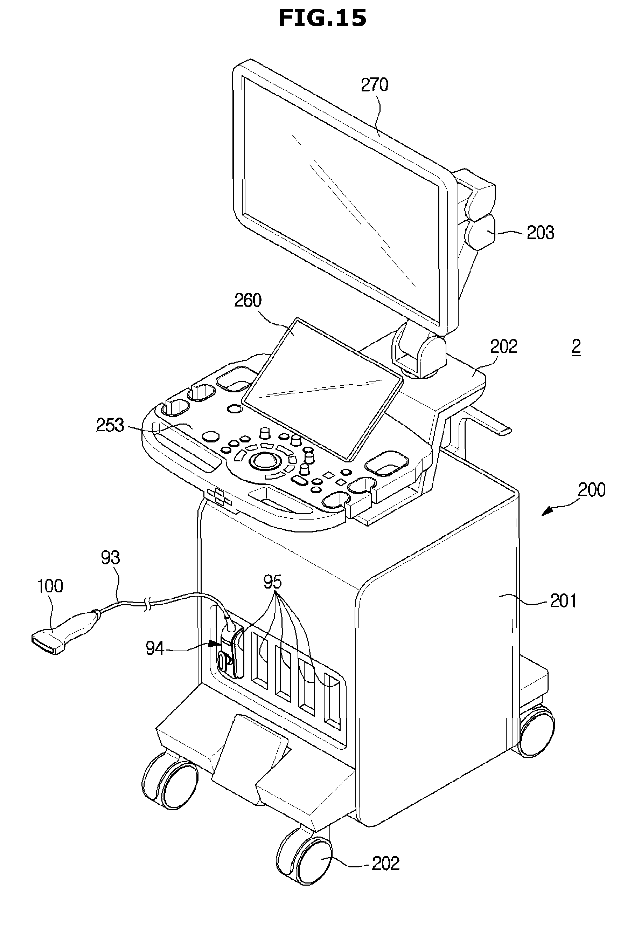

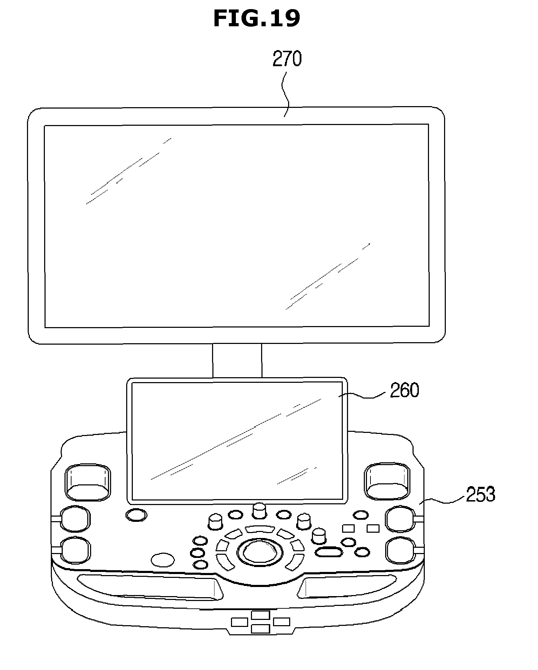

Hereinafter, an ultrasonic imaging apparatus to which the imaging apparatus 1 is applied will be described with reference to FIGS. 15 to 19.

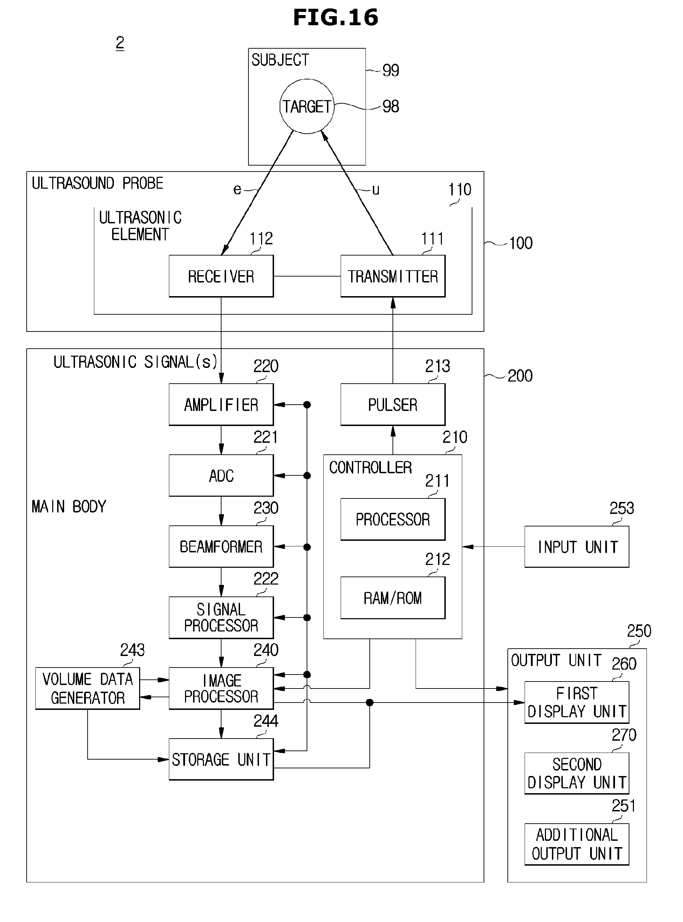

FIG. 15 shows an external appearance of an ultrasonic imaging apparatus according to an embodiment of the present disclosure, and FIG. 16 is a block diagram of an ultrasonic imaging apparatus according to an embodiment of the present disclosure.

As shown in FIG. 16, an ultrasonic imaging apparatus 2 according to an embodiment of the present disclosure may include: an ultrasound probe 100 to receive ultrasonic waves reflected from a target 98 in a subject 99; and a main body 200 to create an ultrasound image using signals output from the ultrasound probe 100 or to generate control signals for controlling the ultrasound probe 100 or various elements installed in the main body 13 according to a user's instruction.

The subject 99 may be an object whose internal structure can be photographed by ultrasonic waves. The subject 99 may be a human body, a fetus, an animal, an object such as a machine or equipment, or the surface of the earth, whose inside can be imaged using ultrasonic waves. The target 98 may be an internal material or structure of the subject 99. The target 98 may reflect ultrasonic waves irradiated from the outside, or generate ultrasonic waves by incident laser.

According to an embodiment, the ultrasound probe 100 and the main body 200 may be connected through a connection cable 93 (see FIG. 15) so that electrical signals output from the ultrasound probe 100 can be transferred to the main body 200 or electrical signals created by the main body 200 can be transferred to the ultrasound probe 100.

In one end of the connection cable 93, a connector 94 may be provided which can be detachably coupled with one of a plurality of ports 95 formed in an external housing 201 of the main body 200. According to an embodiment, the ultrasound probe 100 may be connected to the other end of the connection cable 93. In other words, the ultrasound probe 100 may be integrated with the connection cable 93. According to another embodiment, the other end of the connection cable 93 may include a connector (not shown) that can be detachably coupled with a port installed in the ultrasound probe 100.

According to still another embodiment, the ultrasound probe 100 and the main body 200 may be configured to transfer electrical signals output from the ultrasound probe 100 to the main body 200 or electrical signals generated by the main body 200 to the ultrasound probe 100 through a wireless communication network. In this case, a wireless communication module including an antenna and a wireless communication chip may be installed in each of the ultrasound probe 100 and the main body 200.

The wireless communication module may be a short-range wireless communication module using at least one of Bluetooth, Bluetooth low energy, Infrared Data Association (IrDA), Wireless Fidelity (Wi-Fi), Wi-Fi Direct, Ultra Wideband (UWB), and Near Field Communication (NFC). Also, the wireless communication module may support a 3GPP-based, 3GPP2-based, or IEEE-based wireless communication network authenticated by the International Telecommunication Union (ITU).

The ultrasound probe 100 may receive ultrasonic waves e generated by the target 98 in the subject 99. According to an embodiment, the ultrasound probe 100 may generate ultrasonic waves u, and transmit the ultrasonic waves u to the target 98 in the subject 99.

Referring to FIG. 16, the ultrasound probe 100 may include an ultrasonic element 110 to receive ultrasonic waves e and output ultrasonic signals s being electrical signals corresponding to the received ultrasonic waves e. The ultrasonic element 110 may generate ultrasonic waves u and irradiate the ultrasonic waves u to the target 98. The ultrasound probe 100 may include a plurality of ultrasonic elements 110.

The ultrasonic element 110 may include a transmitter 111 and a receiver 112. The transmitter 111 may vibrate according to the frequency of an electrical signal applied from a pulser 213 to generate ultrasonic waves u of a frequency corresponding to the vibration frequency. The receiver 112 may vibrate according to a frequency of ultrasonic waves e transferred from the target 98 to output an ultrasonic signal s of a frequency corresponding to the vibration frequency. According to an embodiment, the ultrasonic element 110 may be a transmission/reception device (not shown) that can perform both transmission and reception of ultrasonic waves.

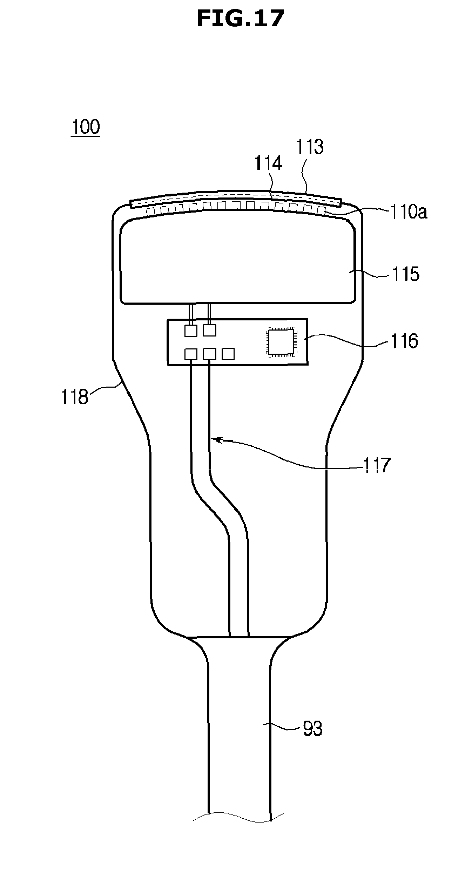

Hereinafter, the ultrasound probe 100 will be described in more detail with reference to FIG. 17. FIG. 17 is a cross-sectional view of an ultrasound probe according to an embodiment of the present disclosure.

Referring to FIG. 17, an ultrasound probe 100 may include an acoustic lens 113, an acoustic matching layer 114, a plurality of ultrasonic elements 110a, an ultrasonic element support 115, a board 116, a conducting wire 117, and an ultrasound probe housing 118 in which the above-mentioned components are installed.

The acoustic lens 113 may focus or diverge ultrasonic waves u passing therethrough. According to an embodiment, the acoustic lens 113 may refract ultrasonic waves u passing therethrough so as to focus the ultrasonic waves u on the target 98. The acoustic lens 113 may be in a curved shape in order to focus or diverge ultrasonic waves u. The acoustic lens 113 may be fabricated with glass or synthetic resins.

The acoustic matching layer 114 may offer a function of maintaining the straightness, wave-form characteristic, and intensity of ultrasonic waves generated by the ultrasonic elements 110, or a function of minimizing reflection of ultrasonic waves from medium. The acoustic matching layer 114 may be positioned adjacent to the acoustic lens 113. The acoustic matching layer 114 may be made of metal powder, ceramic powder, or a silicon wafer.

The ultrasonic elements 110 may convert electrical signals of a predetermined frequency into mechanical vibrations of the same frequency to generate ultrasonic waves of a frequency corresponding to the frequency of the electrical signals. More specifically, if a voltage generated by the pulser 213 is applied to the ultrasonic elements 110, the piezoelectric vibrators or thin films of the ultrasonic elements 110 may vibrate, and ultrasonic waves may be generated from the ultrasonic elements 110 according to the vibrations of the piezoelectric vibrators or thin films. Accordingly, the ultrasonic elements 110 may generate ultrasonic waves. The ultrasonic waves generated by the ultrasonic elements 110 may be focused on the target 98 in the subject 99. The ultrasonic waves may be focused on a target (single focusing), or on a plurality of targets (multi-focusing).

Also, the ultrasonic elements 110 may output ultrasonic signals s, while receiving ultrasonic waves and vibrating at a frequency corresponding to the frequency of the received ultrasonic waves. Since an ultrasonic element 110 can output a signal of a channel, the plurality of ultrasonic elements 110 may output signals of multiple channels. The output ultrasonic signals s may be transferred to an amplifier 220 or a beamformer 230.

The ultrasonic elements 110 may be implemented using ultrasonic transducers. The ultrasonic transducer may be a piezoelectric ultrasonic transducer using the piezoelectric effect of a piezoelectric material, a magnetostrictive ultrasonic transducer using the magnetostrictive effect of a magnetic material, or a capacitive micromachined ultrasonic transducer (CMUT) using vibration of several hundreds or thousands of micromachined thin films. However, the ultrasonic transducer may be any other type ultrasonic transducer capable of generating ultrasonic waves according to electrical signals or generating electrical signals according to ultrasonic waves.

The ultrasonic elements 110 may be installed on the front part of the ultrasonic element support 115. The front part of the ultrasonic element support 115 is one side of the ultrasonic element support 115 facing the subject 99. The ultrasonic elements 110 may be arranged in various ways on the front part of the ultrasonic element support 115. If the ultrasound probe 100 is a 1 Dimensional (1 D) array ultrasound probe, the ultrasonic elements 110 may be arranged in a line on the ultrasonic element support 115. If the ultrasound probe 100 is a 2D array ultrasound probe, the ultrasonic elements 110 may be arranged in a plurality of lines on the ultrasonic element support 115.

The ultrasonic element support 115 may support the ultrasonic elements 110, and absorb ultrasonic waves irradiated in a direction that is opposite to the direction toward the subject 99 among ultrasonic waves generated from the ultrasonic elements 110, or emit heat generated during operation of the ultrasonic elements 110. The ultrasonic element support 150 may be made of a sound absorption material to absorb ultrasonic waves or a heat transfer material to help emission of heat. The sound absorption material may include an epoxy resin or hafnium oxide, and the heat transfer material may include graphite, tungsten, tungsten oxide, silicon, aluminum oxide, and the like.

In the back or side of the ultrasonic element support 115, the board 116 on which an ultrasound probe processor is mounted may be provided. The ultrasound probe processor may perform various signal processing on ultrasonic signals s, or generate control signals for controlling the overall operations of the ultrasound probe 10. The conducting wire 117 extending from the cable 93 may be connected to the board 116.

The external housing 118 may install various components therein, and include a handle that can be gripped by a user. The external housing 118 may have a predetermined shape according to the kind of the subject 99 or the target 98. For example, the external housing 118 may have a shape that is suitable for a specific ultrasonic imaging apparatus, for example, an abdomen ultrasonic imaging apparatus, a vagina ultrasonic imaging apparatus, an anus ultrasonic imaging apparatus, or a kidney ultrasonic imaging apparatus.

A user may grip the external surface of the external housing 118, and cause the front part of the ultrasound probe 100 in which the ultrasonic elements 110a are installed to face the subject 99 so that the ultrasound probe 100 can irradiate ultrasonic waves u to the target 98 in the subject 99 or can receive ultrasonic waves e generated by or reflected from the target 98.

Referring to FIG. 16, the main body 200 may include a controller 210, the pulser 213, the amplifier 220, an Analog-to-Digital Converter (ADC) 221, the beamformer 230, a signal processor 222, an image processor 240, a volume data generator 243, and a storage unit 244. However, some of the above-mentioned components may be omitted as necessary. Also, some of the components may be installed in the ultrasound probe 100 or in a separate workstation (not shown) connected to the main body 200 through a wired/wireless communication network.

The controller 210, the pulser 213, the amplifier 220, the ADC 221, the beamformer 230, the signal processor 222, the image processor 240, and the volume data generator 243 may be implemented by a processor (for example, at least one of a CPU and a GPU) installed in the ultrasound probe 100, the main body 200, or a workstation. The CPU and the GPU may be implemented by various kinds of semiconductor chips and a Printed Circuit Board (PCB) on which the semiconductor chips are mounted.

The controller 210 may control the overall operations of the ultrasonic imaging apparatus 2 according to a user's instruction or a predetermined setting. For example, the controller 210 may control the pulser 213 to control irradiation of ultrasonic waves of the ultrasound probe 100. The controller 210 may generate a control signal according to a frequency of ultrasonic waves that are to be irradiated, and transfer the control signal to the pulser 213. The control signal transferred to the pulser 213 may include information about the frequency or magnitude of a voltage that is to be applied to the transmitter 111. As another example, the controller 210 may control a screen that is displayed on a first display unit 260. More specifically, the controller 210 may determine whether the first display unit 260 outputs an operation menu screen to provide a user with operation menus or outputs a virtual keyboard to provide the user with the virtual keyboard.

According to an embodiment, the controller 210 may include a processor 211, and Random Access Memory (RAM)/Read Only Memory (ROM) 212. The processor 211 may perform various operations required for operations of the ultrasonic imaging apparatus 2, and generate control signals for controlling operations of the ultrasound probe 100 and the main body 200. The processor 211 may be a predetermined algorithm programmed to perform various operations and control operations. The processor 211 may be implemented with one or more semiconductor chips and their components. The RAM/ROM 212 may temporarily or semipermanently store programs related to the processor 211, or may temporarily or non-temporarily store data transferred from the input unit 253 or the first display unit 260 implemented as a touch screen to thereby support the operations of the processor 211.

The pulser 213 may generate a voltage for driving the ultrasonic elements 110 of the ultrasound probe 100. The ultrasonic elements 110 may vibrate according to the amplitude and frequency of a voltage that is output from the pulser 213 to generate ultrasonic waves. The frequency and intensity of ultrasonic waves generated by the ultrasonic elements 110 may depend on the amplitude and frequency of the voltage generated by the pulser 213. The voltage output from the pulser 213 may be applied to the ultrasonic elements 110 at predetermined time intervals, and accordingly, ultrasonic waves generated by the ultrasonic elements 110 may be focused at a predetermined location or directed in a predetermined direction.

As described above, the ultrasonic elements 110 may irradiate ultrasonic waves to the target 98 in the subject 99, and the target 98 may reflect the irradiated ultrasonic waves. The reflected ultrasonic waves e may be received by the plurality of ultrasonic elements 110. The ultrasonic elements 110 may output ultrasonic signals s of multiple channels according to the received ultrasonic waves e, and the ultrasonic signals s of multiple channels may be transferred to the amplifier 220.

The amplifier 220 may amplify the ultrasonic signals s of multiple channels output from the ultrasonic elements 110. A gain of the amplifier 220 may be arbitrarily decided by a system designer or a user of the ultrasonic imaging apparatus 2. According to an embodiment, the amplifier 220 may amplify the ultrasonic signals s of multiple channels output from the plurality of ultrasonic elements 110 to different magnitudes, thereby compensating for differences in intensity between the ultrasonic signals s of multiple channels.

The ADC 221 may convert the ultrasonic signals s being analog signals into digital signals, and then transfer the digital signals to the beamformer 230. The ADC 221 may perform sampling on the ultrasonic signals s being analog signals at a predetermined sampling rate to output digital signals.

The beamformer 230 may focus the ultrasonic signals s of multiple channels. The beamformer 230 may focus signals transferred from the ultrasonic elements 110, the amplifier 220, or the ADC 221 to generate a beamformed signal. The beamformer 230 may perform electronic beam scanning, steering, focusing, apodizing, and an aperature function on the signals s of multiple channels.

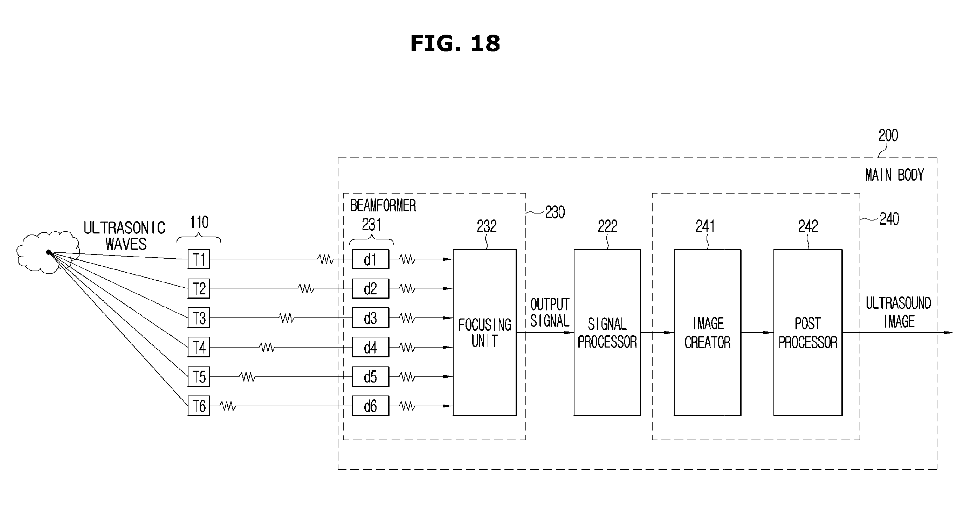

FIG. 18 is a view for describing a beamforming process.

According to an embodiment, the beamformer 230 may include a time difference corrector 231 and a receiver focusing unit 232, as shown in FIG. 18.

The time difference corrector 231 may correct time differences between the ultrasonic signals s of multiple channels. The ultrasonic signals s of multiple channels output from the plurality of ultrasonic elements 110 may have time differences according to distances between the target 98 and the individual ultrasonic elements 110 or according to the properties of the ultrasonic elements 110. The time difference corrector 231 may delay transmission of some of the signals s of multiple channels to correct time differences between the signals s of multiple channels. The receiver focusing unit 232 may synthesize the ultrasonic signals s of multiple channels subject to time difference correction by the time difference corrector 231 to generate a beamformed signal.

The receiver focusing unit 232 may apply predetermined weights to the ultrasonic signals of the individual channels, respectively, to synthesize the ultrasonic signals s of multiple channels. The predetermined weights may be decided regardless of the ultrasonic signals or depending on the ultrasonic signals. The beamformed signal may be transferred to the signal processor 222.

The amplifier 220, the ADC 221, and the beamformer 230 may be implemented as a processor that can be configured with a semiconductor chip, etc. installed in the ultrasound probe 100.

The signal processor 222 may perform various signal processing on the beamformed signal. For example, the signal processor 222 may perform at least one of filtering, detection, and compression on the beamformed signal. The filtering may apply a filter to the beamformed signal to remove other signals except for a signal of a specific bandwidth. The filtering may include a harmonic imaging process of removing fundamental frequency components and passing harmonic signals. The detection may be a process of converting a voltage of an ultrasonic signal in the form of a radio frequency into the format of a video signal. The compression may be a process of reducing differences in amplitude between ultrasonic signals. However, the signal processor 222 may be omitted as necessary.

The image processor 240 may convert the beamformed signal or the signal processed by the signal processor 222 into an ultrasound image in the form of a still image or a moving image, and also may perform predetermined image processing on the still image or the moving image as necessary. The image processor 240 may include an image creator 241 and a post processor 242, as shown in FIG. 18.

The image creator 241 may use scan conversion to create an ultrasound image. The ultrasound image may be an A-mode, B-mode, or M-mode ultrasound image. The A-mode ultrasound image is an ultrasound image obtained by imaging a degree of reflection as an amplitude based on a distance or a time of arrival of ultrasonic waves between the target 98 and the ultrasound probe 100. The B-mode ultrasound image is an ultrasound image obtained by representing an intensity of ultrasonic waves using brightness. The M-mode ultrasound image is an ultrasound image obtained by imaging a degree of change in motion of a subject. Also, the ultrasound image may be a Doppler image using the Doppler effect.

The post processor 242 may correct the created ultrasound image. For example, the post processor 242 may correct the brightness, luminance, sharpness, contrast, or colors of the entire or a part of the ultrasound image so that a user can clearly see tissue in the ultrasound image. The post processor 242 may remove noise or perform interpolation.

The image processor 240 may transfer the created or corrected ultrasound image to the storage unit 244 to store the ultrasound image, or transfer the ultrasound image to the second display unit 270 of the output unit 250 to display the ultrasound image. Also, the image processor 240 may transfer the created or corrected ultrasound image to the volume data generator 243 to acquire ultrasonic volume data.

The volume data generator 243 may acquire ultrasonic volume data representing a 3D volume using a 2D ultrasound image created or corrected by the image processor 240.

In FIG. 16, an embodiment in which the ultrasonic imaging apparatus 2 includes the input unit 253, the first display unit 260 implemented as a touch screen, and the second display unit 270 is shown.

The output unit 250 may include the first display unit 260 to display a virtual keyboard or operation menus required for controlling the ultrasonic imaging apparatus 2, and the second display unit 270 to display ultrasound images or ultrasonic volume data.

As shown in FIGS. 15 and 16, the first display unit 260 and the second display unit 270 may be separated from each other, and mounted on the main body 200. The first display unit 260 may be placed at a position at which a user can easily perform a touch operation. For example, the first display unit 260 may be mounted on the input unit 253. The second display unit 270 may be placed at a user's eye level so that the user can easily see ultrasound images. For example, the second display unit 270 may be placed at a higher position than the first display unit 260 through one or more support frames 202 and 203 extending from the external frame 201 of the main body 200.

The first display unit 260 may be a touch screen. In this case, the user may touch a guide image displayed on the first display unit 260 to input a desired command to the ultrasonic imaging apparatus 2. The second display unit 270 may be a touch screen or not. The first display unit 260 and the second display unit 270 may be implemented using a CRT, a PDP, LEDs, a LCD, a QD-LED display, or E-Ink.

The first display unit 260 may display an operation menu screen for controlling the ultrasonic imaging apparatus 2, and a virtual keyboard screen.

For example, the first display unit 260 may display the operation menu screen, and when a virtual keyboard call command is input through the input unit 253 or the first display unit 260, the first display unit 260 may display the virtual keyboard screen. In this case, a virtual keyboard may overlap the operation menu screen to block various guide images displayed on the operation menu screen.

When the virtual keyboard is displayed, the first display unit 260 may further display a sub menu. Accordingly, even when the virtual keyboard is displayed to block the operation menus of the operation menu screen, a user can input a desired command using the sub menu. That is, even when it is difficult to manipulate the operation menus due to the virtual keyboard, a user can quickly input a command for controlling the ultrasonic imaging apparatus 2 using the sub menu. The operation menu screen, the virtual keyboard, the sub menu, and the real-time information display area have been described above, and accordingly, further descriptions thereof will be omitted.

The output unit 250 may further include an additional output unit 251. The additional output unit 251 may output various information related to the ultrasonic imaging apparatus 2 in the form of sound or light, in addition to the first display unit 260 and the second display unit 270. The additional output unit 250 may include a speaker or a lighting device such as a LED.

The input unit 253 may output an electrical signal according to the user's manipulation. The electrical signal may be transferred to the controller 210. The controller 210 may generate a control signal corresponding to the received electrical signal, and transfer the control signal to the individual components of the ultrasonic imaging apparatus 2. Accordingly, the ultrasonic imaging apparatus 2 may receive various commands related to control operations of the ultrasonic imaging apparatus 2 from the user. The input unit 253 may include a mouse, a physical keyboard, a physical button, a track ball, a touch pad, a stick type manipulation unit, or a knob.

According to an embodiment, the input unit 253 may receive a virtual keyboard call command. Also, the input unit 253 may allow the user to select a guide image of the operation menus, a key of the virtual keyboard, or a guide image of the sub menu.

So far, an example in which the imaging apparatus 1 is applied to the ultrasonic imaging apparatus 2 has been described. However, the imaging apparatus 1 may be applied to any other apparatus that can be considered by one of ordinary skill in the art, for example, a visible light camera, an infrared camera, a camcorder, a digital radiation imaging apparatus, a CT apparatus, a Mammography apparatus, and a MRI apparatus in the same manner or through appropriate modifications.

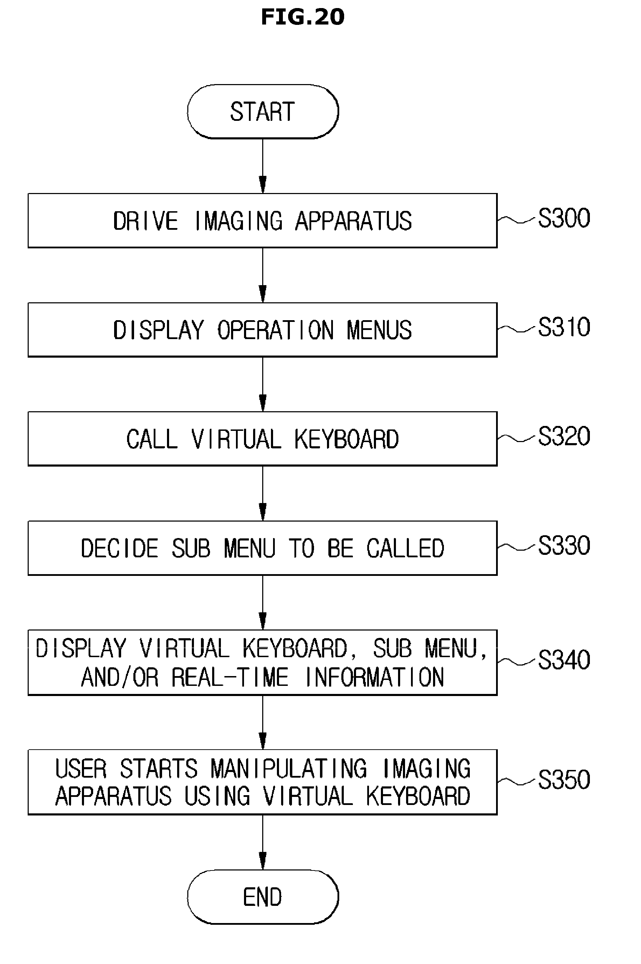

Hereinafter, a method of controlling an imaging apparatus, according to an embodiment of the present disclosure, will be described with reference to FIG. 20.

FIG. 20 is a flowchart illustrating a method of controlling an imaging apparatus, according to an embodiment of the present disclosure.

As shown in FIG. 20, an imaging apparatus may operate, in operation S300. If the imaging apparatus operates, at least one application may be executed according to a user's manipulation or a predetermined programming. The application may be to perform at least one of various functions of the imaging apparatus. The application may be executed at the time when the imaging apparatus operates or after the imaging apparatus operates.

If the imaging apparatus operates, a first display unit mounted on the imaging apparatus and configured to display images may display operation menus using an operation menu screen, in operation S310. The operation menus may include guide images, such as various operation buttons or track bars, for manipulating the imaging apparatus. If the first display unit is a touch screen, a user may touch one of the guide images displayed on the screen to select at least one function of a plurality of functions corresponding to the operation menus. If the first display unit is not a touch screen, the user may use separate input means, such as a mouse or a track ball to select one of the guide images displayed on the screen to select at least one function of the plurality of functions corresponding to the operation menus.

The imaging apparatus may call a virtual keyboard according to the user's manipulation or a predetermined setting, in operation S320. For example, if the user touches a guide image related to a virtual keyboard call function among the operation menus or selects the guide image using input means such as a mouse, the imaging apparatus may call a virtual keyboard. As another example, when a situation needing to input a character or a numeral using a virtual keyboard occurs, the imaging apparatus may call a virtual keyboard automatically.

If the virtual keyboard is called, a sub menu that is to be displayed on the first display unit together with the virtual keyboard before or after the virtual keyboard is displayed may be decided, in operation S330. The sub menu may be decided according to the user's setting or a predetermined setting.

The sub menu, which relates to the operation menus, may be configured with the entire or a part of functions of the operation menus. For example, the sub menu that is to be displayed on the first display unit may include one or more functions that a user often uses or can often use among the functions of the operation menus. Also, the sub menu may be decided according to an application that is currently executed on the imaging apparatus. The functions that the user often uses or can often use may include one or more functions that the user often uses or can often use when the virtual keyboard is called.

If the sub menu is decided, the virtual keyboard may overlap the operation menus, in operation S340. The virtual keyboard may block the entire area of the operation menus. Accordingly, the user cannot see any operation menus formed on the operation menu screen. Also, the sub menu may be further displayed together with the virtual keyboard. According to an embodiment, a real-time information display area may be further displayed together with the virtual keyboard.

If the virtual keyboard is called and displayed on the first display unit, the user may input a character, a numeral, or a symbol using the virtual keyboard, in operation S350. Accordingly, the user can input various commands using the sub menu even when he/she cannot see the operation menus.

The virtual keyboard and the sub menu may be removed from the screen according to the user's selection or a predetermined setting such as the elapse of a predetermined time. In this case, instead of the virtual keyboard, the operation menus may be displayed on the screen of the first display unit. In other words, only the operation menu may be displayed on the screen.

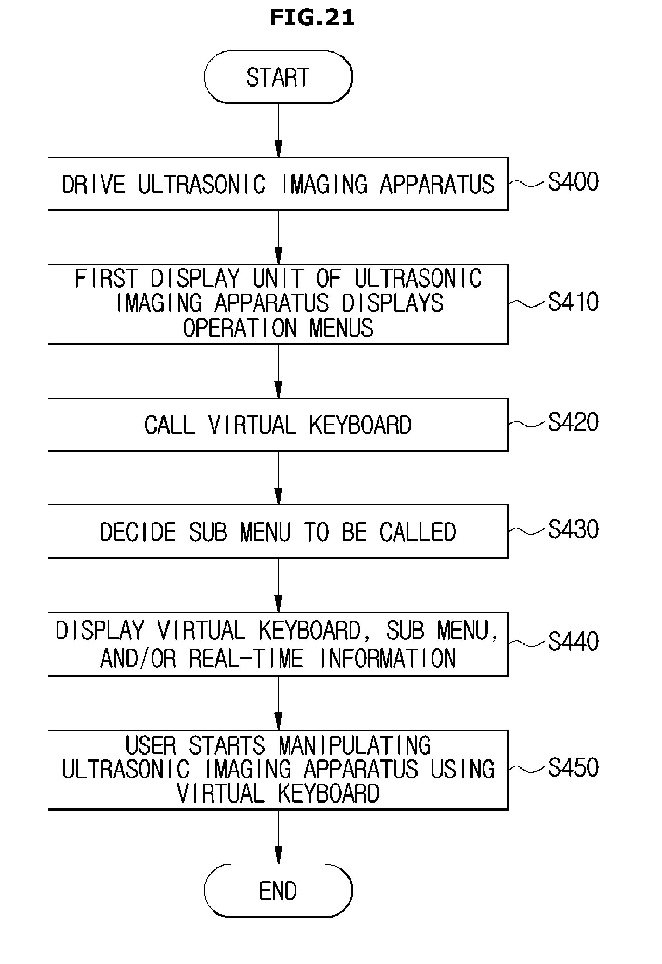

Hereinafter, a method of controlling an ultrasonic imaging apparatus, according to an embodiment of the present disclosure, will be described with reference to FIG. 21.

FIG. 21 is a flowchart illustrating a method of controlling an ultrasonic imaging apparatus, according to an embodiment of the present disclosure. The ultrasonic imaging apparatus may include a plurality of display units, that is, the first display unit 260 and the second display unit 270, as shown in FIGS. 15 and 16.

Referring to FIG. 21, first, the ultrasonic imaging apparatus may operate, and an application related to the ultrasonic imaging apparatus may be executed, in operation S400.

If the ultrasonic imaging apparatus operates, operation menus may be displayed on the first display unit, in operation S410. The operation menus may be displayed in an operation menu screen, and the first display unit may display the operation menu screen to provide the operation menus to a user.

The operation menus may further include a TGC area.

The ultrasonic imaging apparatus may call a virtual keyboard according to the user's manipulation or a predetermined setting, in operation S420.

If the virtual keyboard is called, a sub menu that is to be displayed on the first display unit together with the virtual keyboard may be decided, in operation S430.

The sub menu may be decided according to the user's setting or a predetermined setting. As described above, the sub menu may be configured with the entire or a part of the functions of the operation menus. If the sub menu is configured with a part of the functions of the operation menus, the sub menu may include one or more functions that the user often uses or can often use among the plurality of functions of the operation menus, or the sub menu may include one or more functions corresponding to an application that is currently executed on the ultrasonic imaging apparatus.

According to an embodiment, the sub menu may further include a TGC area as shown in FIG. 4.

If the sub menu is decided, the virtual keyboard may overlap the operation menus, in operation S440. The sub menu may be further displayed together with the virtual keyboard. According to an embodiment, a real-time information display area may be further displayed together with the virtual keyboard.

Then, the user may use the virtual keyboard to input a character, a numeral, or a symbol to manipulate the ultrasonic imaging apparatus, in operation S450.

The virtual keyboard and the sub menu may be removed from the screen according to the user's selection or a predetermined setting. In this case, instead of the virtual keyboard and the sub menu, the operation menus may be displayed on the screen of the first display unit.

According to the imaging apparatus and the control method thereof as described above, even when the virtual keyboard overlaps the operation menus, a user can easily check and execute the operation menus blocked by the virtual keyboard and not seen, which leads to convenience of manipulation.

According to the imaging apparatus and the control method thereof as described above, even when the virtual keyboard is enlarged on the display screen, a user can easily check and execute operation menus as well as the virtual keyboard.

According to the imaging apparatus and the control method thereof as described above, a user can easily and quickly use various functions allocated to the operation menus while using the virtual keyboard.

According to the imaging apparatus and the control method thereof as described above, a user can easily select and execute menus that he/she often uses or menus having high frequency of use in association with a current operation even when using the virtual keyboard.

According to the imaging apparatus and the control method thereof as described above, by displaying a predetermined virtual keyboard according to an application being executed, a user can quickly control the operation of the application.

Although a few embodiments of the present disclosure have been shown and described, it would be appreciated by those skilled in the art that changes may be made in these embodiments without departing from the principles and spirit of the disclosure, the scope of which is defined in the claims and their equivalents.

## Claims

1. An imaging apparatus comprising:
a main body;
a display unit configured to display one or more operation menus for controlling the main body; and
a processor configured to control, if a virtual keyboard call command is received, the display unit to display a virtual keyboard and a sub menu related to the operation menus, instead of the operation menus.

2. The imaging apparatus according to claim 1, wherein the processor controls the display unit to display the virtual keyboard that blocks the entire or a part of the operation menus.

3. The imaging apparatus according to claim 1, wherein the sub menu is displayed above or beside the virtual keyboard, or overlaps the virtual keyboard.

4. The imaging apparatus according to claim 1, wherein the sub menu includes all of one or more functions or commands corresponding to the operation menus, wherein the sub menu includes one or more functions or commands having high frequency of use by a user among one or more functions or commands corresponding to the operation menus, or wherein the sub menu includes one or more functions or commands related to an application being executed on the main body upon manipulation, among one or more functions or commands corresponding to the operation menus.

5. The imaging apparatus according to claim 1, wherein when the virtual keyboard is called, the processor controls the display unit to display information acquired in real time by the main body.

6. The imaging apparatus according to claim 1, wherein the virtual keyboard is called according to a user's manipulation or a predetermined setting.

7. The imaging apparatus according to claim 1, wherein the main body comprises at least one of a visible light camera, an infrared camera, a camcorder, an ultrasonic imaging apparatus, a digital radiation imaging apparatus, a computerized tomography (CT) apparatus, a Mammography apparatus, and a Magnetic Resonance Imaging (MRI) apparatus.

8. The imaging apparatus according to claim 1, wherein at least one of the operation menus and the sub menu further comprises a Time Gain Compensation (TGC) area.

9. The imaging apparatus according to claim 1, wherein the main body further comprises a second display unit configured to display an image acquired by the main body.

10. A method of controlling an imaging apparatus including a main body and a display unit, comprising:
at the display unit, displaying one or more operation menus for controlling the main body;
calling a virtual keyboard; and
at the display unit, displaying, if the virtual keyboard is called, the virtual keyboard and a sub menu related to the operation menus, instead of the operation menus.

11. The method according to claim 10, wherein the virtual keyboard blocks the entire or a part of the operation menus.

12. The method according to claim 10, wherein the displaying, if the virtual keyboard is called, the virtual keyboard and the sub menu related to the operation menus, instead of the operation menus, comprises at the display unit, displaying the sub menu above or beside the virtual keyboard or overlapping the sub menu with the virtual keyboard.

13. The method according to claim 11, further comprising deciding the sub menu according to a user's selection or a predetermined setting.

14. The method according to claim 13, wherein the deciding of the sub menu according to the user's selection or the predetermined setting comprises deciding all of one or more functions corresponding to the operation menus or one or more commands related to the functions, as the sub menu, wherein the deciding of the sub menu according to the user's selection or the predetermined setting comprises deciding one or more functions or commands having high frequency of use by the user among one or more functions or commands corresponding to the operation menus, as the sub menu, or wherein the deciding of the sub menu according to the user's selection or the predetermined setting comprises deciding one or more functions or commands related to an application being executed on the main body upon manipulation, among one or more functions or commands corresponding to the operation menus, as the sub menu.

15. The method according to claim 10, wherein the displaying, if the virtual keyboard is called, the virtual keyboard and the sub menu related to the operation menus, instead of the operation menus, comprises displaying information acquired in real time by the main body.
